# EUROPEAN PATENT SPECIFICATION

(11) **EP 3 337 442 B1**
(45) Date of publication and mention of the grant of the patent: **31.05.2023**
(21) Application number: 16835586.5
(22) Date of filing: 11.07.2016
(51) Int. Cl.: A61H 11/02, A61H 11/00, A61F 7/00, A61N 1/36, A61F 7/02, A61F 7/10, A61N 1/04, A43B 3/00, A43B 5/00, A43B 11/00, A43C 11/00

(54) **COMPRESSION DEVICE**
KOMPRESSIONSVORRICHTUNG
DISPOSITIF DE COMPRESSION

(30) Priority: 11.08.2015 US 201514823040
(43) Date of publication of application: 27.06.2018
(60) Divisional application: 20167068.4
(73) Proprietor: Recovery Force, LLC, Fishers, IN 46038 (US)
(72) Inventor: WYATT, Matthew, W., Fishers, IN 46037 (US); STASEY, Brian, J., Fishers, IN 46038 (US); GUMMIN, Mark, Silverton, OR 97381 (US)
(74) Representative: Algemeen Octrooi- en Merkenbureau B.V.
(86) International application number: PCT/US2016/041706
(87) International publication number: WO 2017/027145

(56) References cited:
- WO-A1-94/09728
- WO-A1-02/054900
- WO-A1-2014/172248
- WO-A2-2013/191933
- DE-A1- 19 833 801
- GB-A- 2 504 984
- JP-A- 2005 098 241
- US-A- 4 862 878
- US-A- 5 997 465
- US-A- 6 032 387
- US-A- 6 123 681
- US-A1- 2002 100 188
- US-A1- 2006 162 332
- US-A1- 2008 066 272
- US-A1- 2010 050 478
- US-A1- 2012 232 447
- US-A1- 2013 104 429
- US-A1- 2013 184 622
- US-A1- 2013 289 461
- US-A1- 2015 065 930
- US-A1- 2015 065 930
- US-A1- 2015 073 318
- US-A1- 2015 089 839
- US-A1- 2015 282 564
- US-B1- 9 161 878
- US-S- D 473 656

## Description

### Background

The present invention is related the technical field of compression devices.

Blood flow disorders can lead to numerous health and cosmetic problems for people. Relatively immobile patients, such as post-operative patients, the bedridden, and those individuals suffering from lymphedema and diabetes can be prone to deep vein thrombosis (DVT). Post-operative patients are often treated with a DVT cuff during surgery and afterwards for up to 72 hours. Clinicians would prefer to send patients home with DVT cuffs and a treatment regimen to reduce the risk of blood clots. However, patient compliance is often a problem because the traditional DVT cuff renders the patient immobile and uncomfortable during the treatment, which can be an hour or more. Travelers confined to tight quarters during airline travel or long-distance driving, for example, are also particularly at risk for the development of thromboses, or blood clots due to decreased blood flow. Varicose veins are another disorder resulting from problems with patient blood flow. Varicose veins are often a symptom of an underlying condition called venous insufficiency. Normal veins have one-way valves that allow blood to flow upward only to return to the heart and lungs. A varicose vein has valves that are not functioning properly. The blood can flow upwards, but tends to pool in the vein because of valve dysfunction. The varicose veins bulge because they are filled with pooled blood. Although varicose veins are often a cosmetic concern, the condition also causes pain, leg heaviness, fatigue, itching, night cramps, leg swelling, and restless legs at night. Varicose vein disease can be treated with various nonsurgical techniques such as sclerotherapy or endovenous laser treatment (EVLT). In some cases enhanced blood flow is essential for quality of life, such as for those individuals suffering from RVD (peripheral vascular disease) and RLS (restless leg syndrome), or women undergoing reconstructive breast surgery suffering from arm pain and fatigue due to poor blood flow.

For some individuals the condition can also be treated by the nightly use of compression stockings. Compression stockings are elastic stockings that squeeze the veins and stop excess blood from flowing backward. These, and other known devices, tend to only provide an initial compression force at a low level that decreases over time upon continued deformation of the stocking. Moreover, stockings of this type are difficult to put on and take off, particularly for the elderly.

Many athletes, whether professionals or lay persons, suffer from muscle soreness, pain and fatigue after exercise due to toxins and other workout by-products being released. Recent research has shown that compression garments may provide ergogenic benefits for athletes during exercise by enhancing lactate removal, reducing muscle oscillation and positively influencing psychological factors. Some early research on compression garments has demonstrated a reduction in blood lactate concentration during maximal exercise on a bicycle ergometer. Later investigations have shown improved repeated jump power and increased vertical jump height . The suggested reasons for the improved jumping ability with compression garments include an improved warm-up via increased skin temperature, reduced muscle oscillation upon ground contact and increased torque generated about the hip joint. Reaction time is important to most athletes, as well as to race car drivers, drag racers and even fighter pilots. Exercise science and kinesiology experts point to training modules, such as PitFit^{™}, that benefit from acute sensory drills and increased oxygen intake related to increased blood flow. Combined, these results show that compression garments may provide both a performance enhancement and an injury reduction role during exercises provoking high blood lactate concentrations or explosive-based movements.

Research has also shown that compression garments may promote blood lactate removal and therefore enhance recovery during periods following strenuous exercise. In one test, significant reduction in blood lactate levels in highly fit were observed in males wearing compression stockings following a bicycle ergometer test at 110 per cent VO2max. Similar results were obtained in a later study in which a significant reduction in blood lactate concentration and an increased plasma volume was found in twelve elderly trained cyclists wearing compression garments following five minutes of maximal cycling. In another test, wearing compression garments during an 80-minute rest period following the five minutes of maximal cycling were shown to significantly increase (2.1 per cent) performance during a subsequent maximal cycling test. It was suggested that increased removal of the metabolic by-products during intense exercise when wearing compression garments may help improve performance. These results suggest that wearing compression garments during recovery periods following high intensity exercise may enhance the recovery process both during and following intense exercise and therefore improve exercise performance.

Compression devices have also been used during recovery periods for athletes following strenuous activity. These devices are generally limited to the athlete's legs and typically comprise a series of inflatable bladders in a heel-to-thigh casing. An air pump inflates the series of bladders in a predetermined sequence to stimulate arterial blood flow through the athlete's legs. Compression devices of this type are extremely bulky, requiring that the athlete remain generally immobile, either seated or in a prone position.

United States patent application US 2015/065930 A1 discloses a mobile compression integument for applying controllable scrolling or intermittent sequential forces, such as compression forces, to the body and limbs of a user. The compression integument comprises an elongated fabric body sized to encircle a limb of a user, one or more shape-changing elements carried by the fabric body and configured to apply a compression pressure to the limb through the fabric body upon changing shape in response to a stimulus, and a micro-processor based controller for selectively actuating the one or more shape-changing elements to reduce the effective diameter of the integument encircling the limb, to thereby apply pressure to the limb.

United States patent application US 6 032 387 A discloses an automated tightening and loosening shoe with rigid, semi-rigid or flexible polymer bands functioning as laces, and a tightening mechanism which operates in one direction to cause automatic tightening of the rigid, semi-rigid or flexible polymer bands to tighten the automated tightening and loosening shoe about a wearer's foot, and which operates in a reverse or loosening direction to cause automatic loosening of the rigid, semi-rigid or flexible polymer bands so that the automated tightening and loosing shoe can be removed from the wearer's foot.

There is a need for improved devices and associated methods for compressing a portion of a patient's or athlete's body, and even an animal's body, such as a race horse or working dog. Of particular need is a device that is comfortable and mobile. Current technology uses plastic (PVC) wrapped around the extremity causing enhanced perspiration and discomfort, so a device that is comfortable and mobile will increase athlete and patient compliance with a treatment regimen. In patients, such compliance may reduce the risk of DVT and/or related peripheral vascular disease (PVD), or venous flow anomalies which could have positive economic impact on costs of healthcare.

### Summary

In general terms, constrictor devices were developed by vascular surgeons to increase arterial blood flow. These devices apply a massage-like compression to the foot, ankle and calf to circulate blood flow with no known side effects. Current constrictor devices rely upon air pressure from an external air pump to cause constriction compression for patient treatment.

According to this invention the compression device or device is an apparatus that utilizes shape changing materials in conjunction with elongated compression textiles or fabrics to apply controllable intermittent sequential compression or constriction pressure to a body portion of a person, typically an extremity such as the arms or legs. One form of compression pattern is an infinite series of scrolling actions as the compression is successively applied to segments of the patient's limb. The compression device herein is a self-contained unit within a wearable extremity device. An on-board microprocessor controls the constriction of the shape changing materials and an on-board power supply provides the power for the compression actuation. By using this self contained low profile unit, a patient or athlete can remain mobile and compliant with the treatment regiment because of the device's comfort, allowing the user to engage in everyday activities. The device described herein also reduces costs to the use by eliminating the need to rent or purchase a specialized external air pump.

In one aspect, the shape changing material may be a shape memory metal that contracts in response to heat or an electrical current. In another aspect, the shape changing material may be a phase change material that contracts as the material changes phase.

### Description of the Figures

FIG. 1 is a plan view of an device according to one disclosed embodiment.
FIG. 2 is a top view of a circuit board for use in the device shown in FIG. 3.
FIG. 3 is a circuit diagram for the electrical circuit of the device shown in FIG. 3.
FIG. 4 is a top view of a compression device according to yet another aspect of the present disclosure.
FIG. 5 is a view of one face of a strap component of the compression device shown in FIG. 4.
FIG. 6 is a view of an opposite face of the strap component shown in FIG. 27.
FIG. 7 is a cut-away view of the strap component shown in FIGS. 5-6.
FIG. 8 is a top view of an accessory component for use with the compression device shown in FIG. 4.
FIG. 9 is a plan view of the inner assembly of a compression device according to a further aspect of the present disclosure.
FIG. 10 is a plan view of the outer face of the compression device shown in FIG. 9.
FIG. 11 is a plan view of the inner face of the compression device shown in FIGS. 9-10.
FIG. 12A is an enlarged view of a tensioner used in the compression device shown in FIGS. 9-11.
FIG. 12B is an enlarged view of an alternative configuration of the tensioner shown in FIG. 12A.
FIG. 12C is an enlarged view of SMA wires in an alternative configuration of the tensioner shown in FIG. 12B.
FIG. 13 is a perspective view of a full lower body compression device according to one feature of the present disclosure.
FIG. 14 is a perspective view of a single leg compression device according to a further feature of the present disclosure.
FIG. 15 is a perspective view of the single leg compression device of FIG. 14 shown wrapped around a user's leg.
FIG. 16 is a perspective view of an ankle compression device according to the present disclosure.
FIG. 17 is a perspective view of an wrist compression device according to the present disclosure
FIGS. 18A, 18B are perspective views of a shoulder compression device according to the present disclosure.
FIG. 19 is a perspective view of a torso compression device according to the present disclosure.
FIG. 20 are perspective views of a compression device adapted to treat the hip, IT band and gluteus muscles of the wearer according to the present disclosure.
FIG. 22 are perspective views of a lumbar compression device according to the present disclosure.

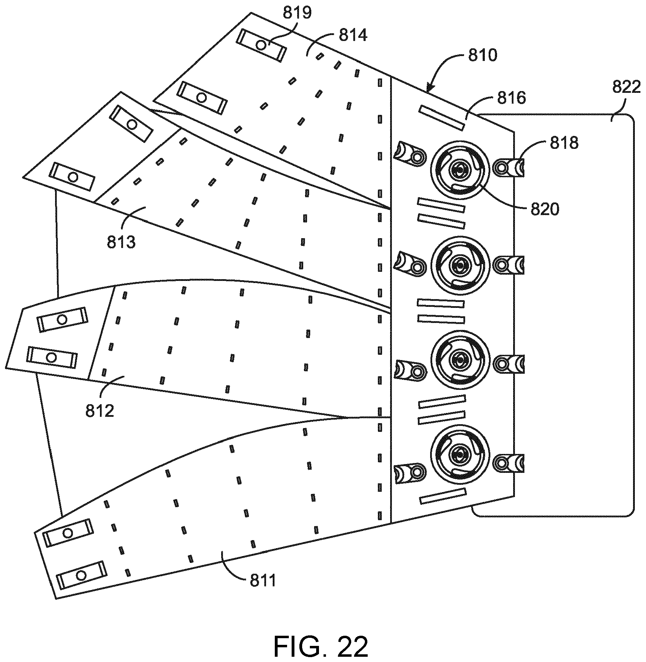

FIG. 22 is a plan view of a calf compression device according to the present disclosure.

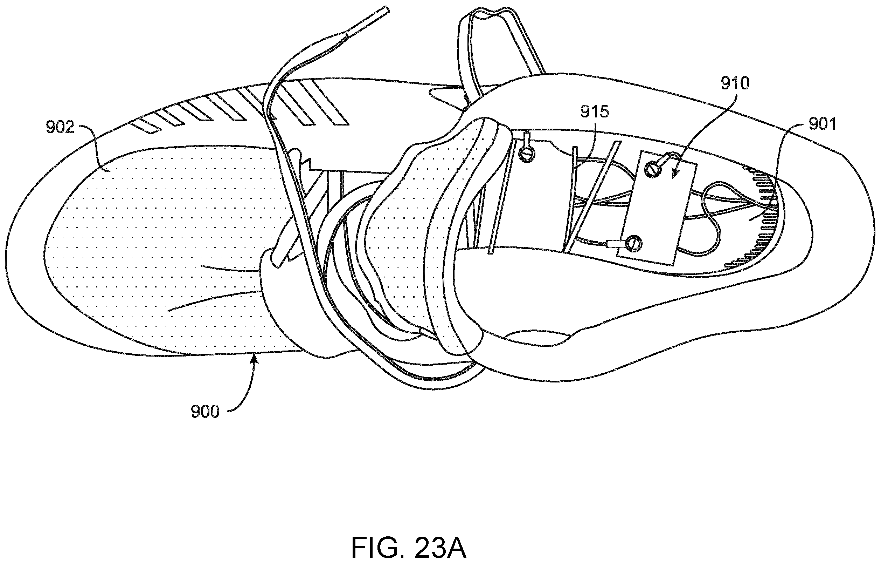

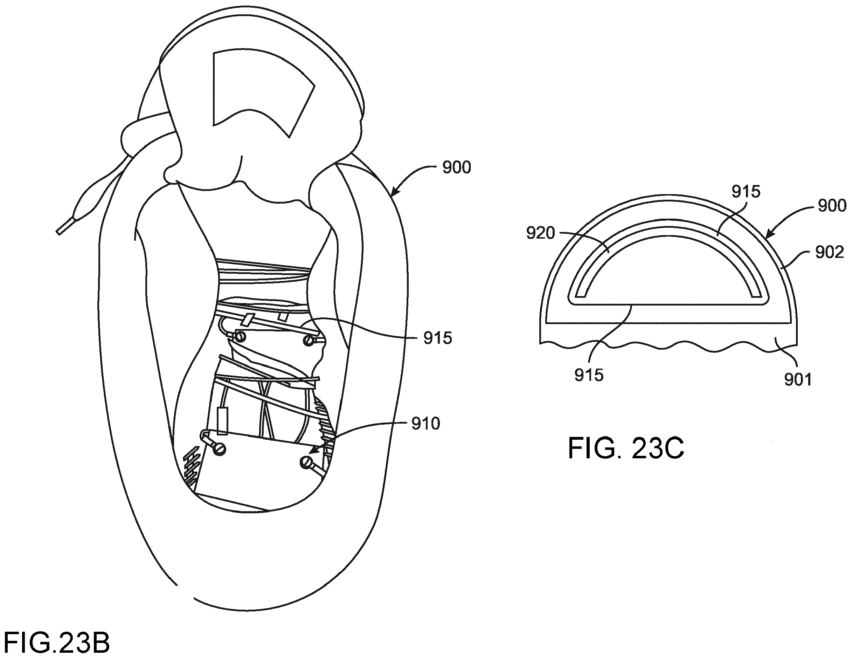

FIGS 23A, 23B are top perspective views of a compression device integrated into a shoe.
FIG. 23C is an end view in cross-section of the show in FIGS. 23A, 23B showing components of the compression device.

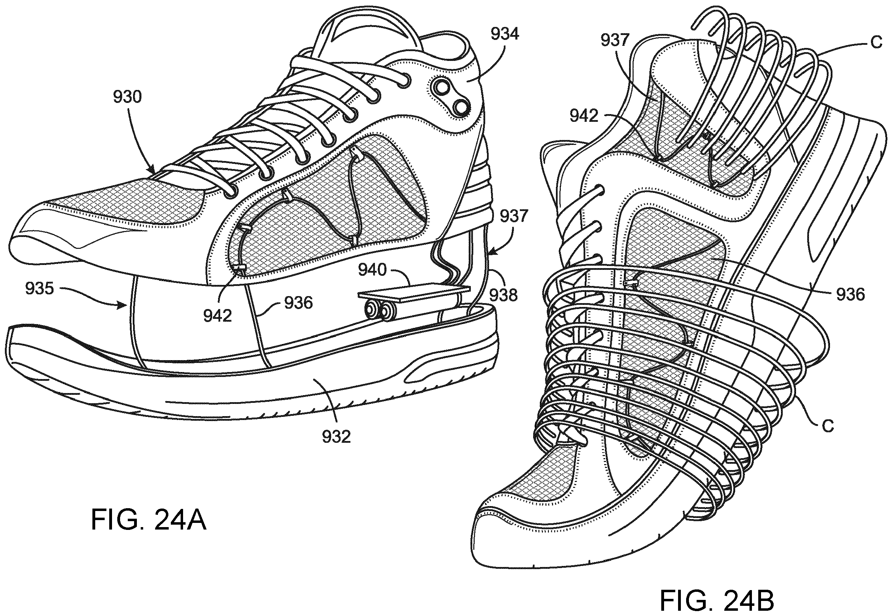

FIG. 24A is perspective view of a compression device implemented within a shoe according to one embodiment of the present disclosure.

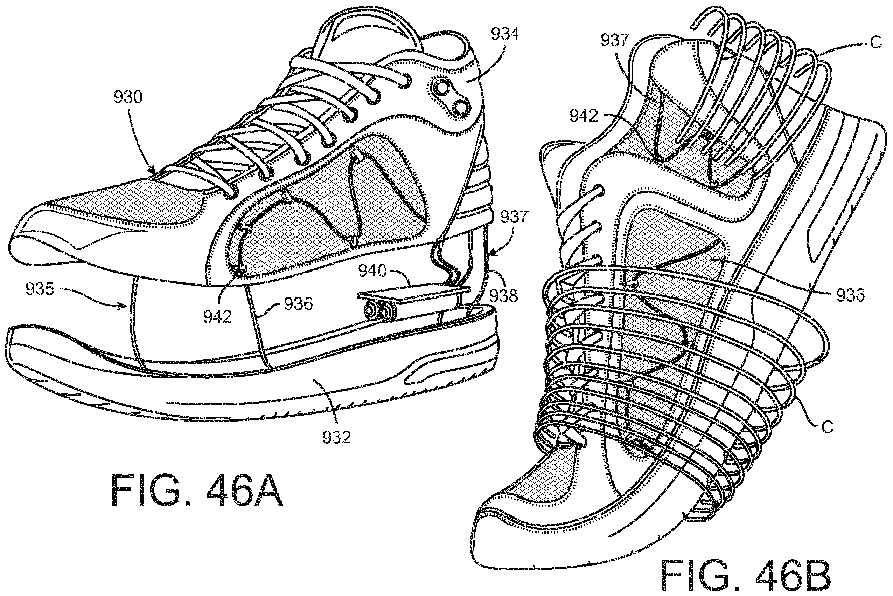

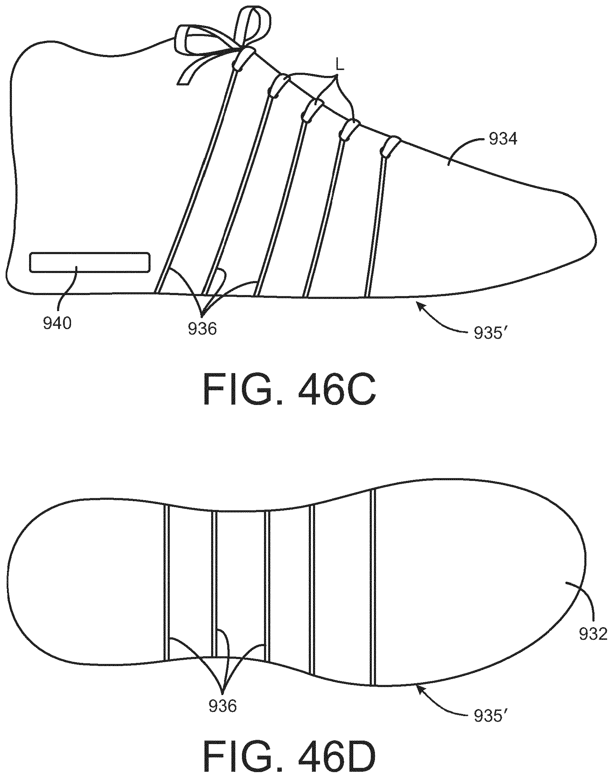

FIG. 24B is a perspective view of the shoe shown in FIG. 46a with a representation of lines of compression force.

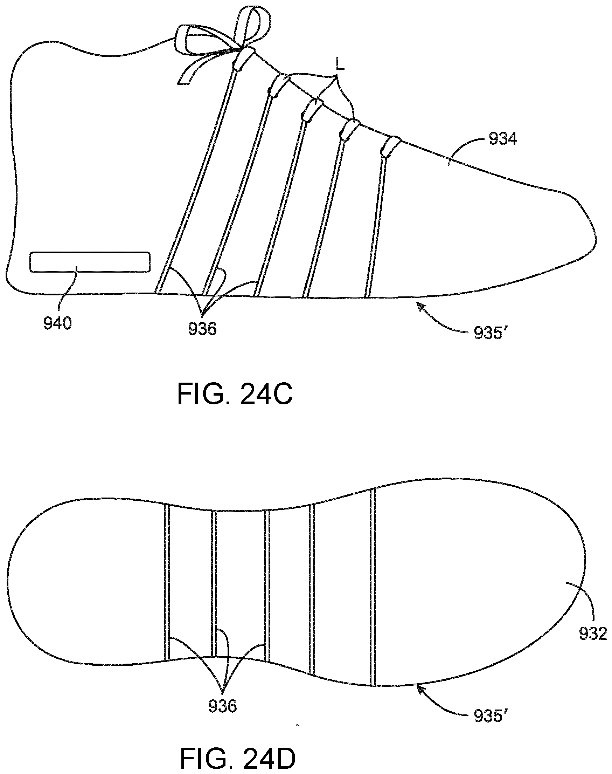

FIGS. 24C, 24D are side and bottom views of a shoe incorporating a compression device with an alternative configuration.

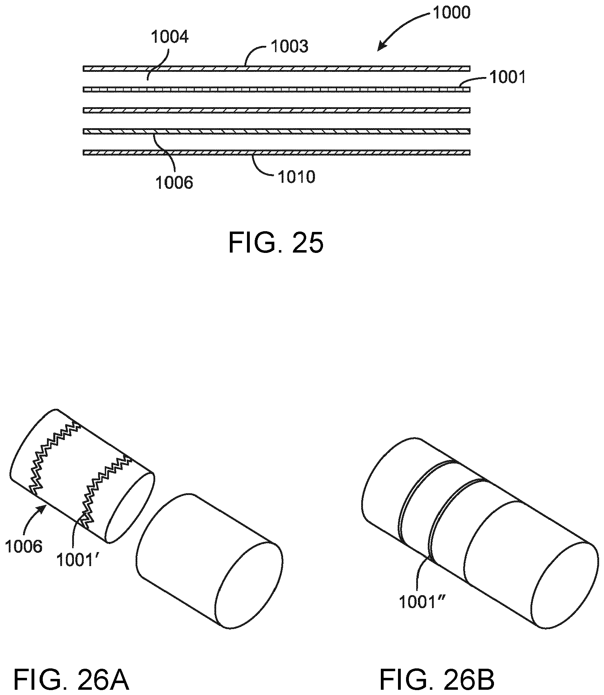

FIG. 25 is a side representation of an SMA wire according to one aspect of the disclosure in which the wire is embedded within a sheath.
FIGS. 26A, 26B are representations of a compression device formed in a sinusoidal configuration with the SMA wire depicted in its initial and stretched states.

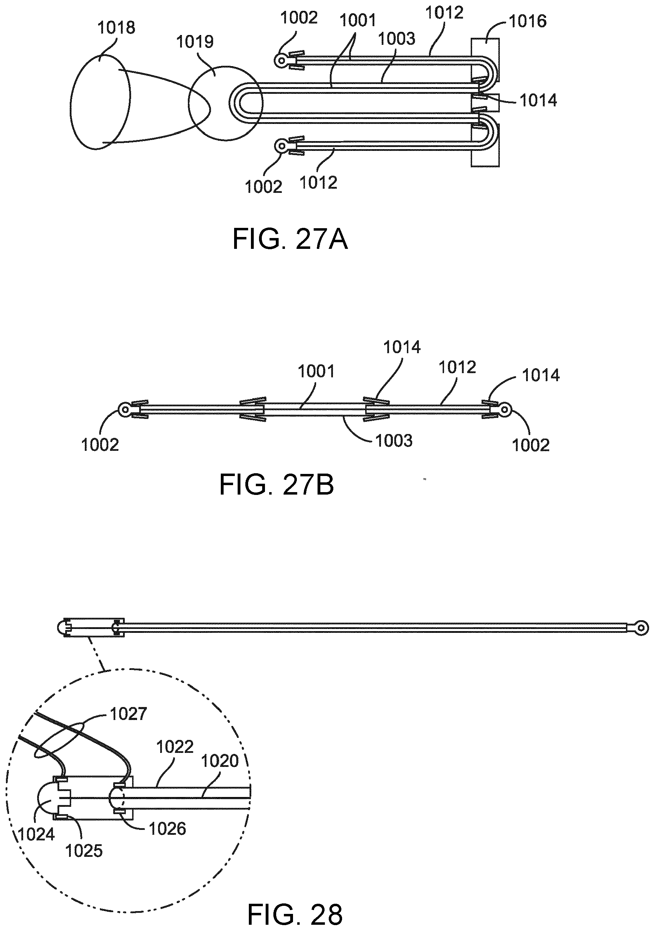

FIGS. 27A, 27B are top and side representation of an SMA wire within a sheath with an anchor according to a further aspect of the present disclosure.
FIG. 28 is a side and enlarged view of an SMA wire anchored to an end connector according to a further aspect of the disclosure.

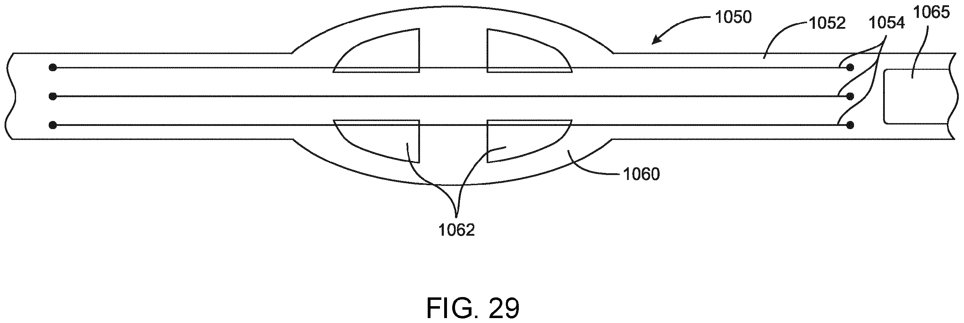

FIG. 29 is a top view of compression device including an electrical stimulation device according to one embodiment of the present disclosure.

### Detailed Description

For the purposes of promoting an understanding of the principles of the invention, reference will now be made to the embodiments illustrated in the drawings and described in the following written specification. It is understood that no limitation to the scope of the invention is thereby intended. It is further understood that the present invention includes any alterations and modifications to the illustrated embodiments and includes further applications of the principles of the invention as would normally occur to one skilled in the art to which this invention pertains.

The present disclosure contemplates a compression device that provides the same efficacy for blood flow circulation improvement afforded by current pneumatic arterial constriction devices, but in a device that is not restrictive to the patient or athlete during a compression treatment. Current products require the patient to remain relatively immobile in a seated position or prone while air bladders in the wrap are inflated and deflated. Inflation and deflation of the air bladders requires a bulky external pump and hoses, which effectively ties the user to one location. The present invention contemplates a device that can be easily and comfortably worn while allowing full mobility of the patient or athlete.

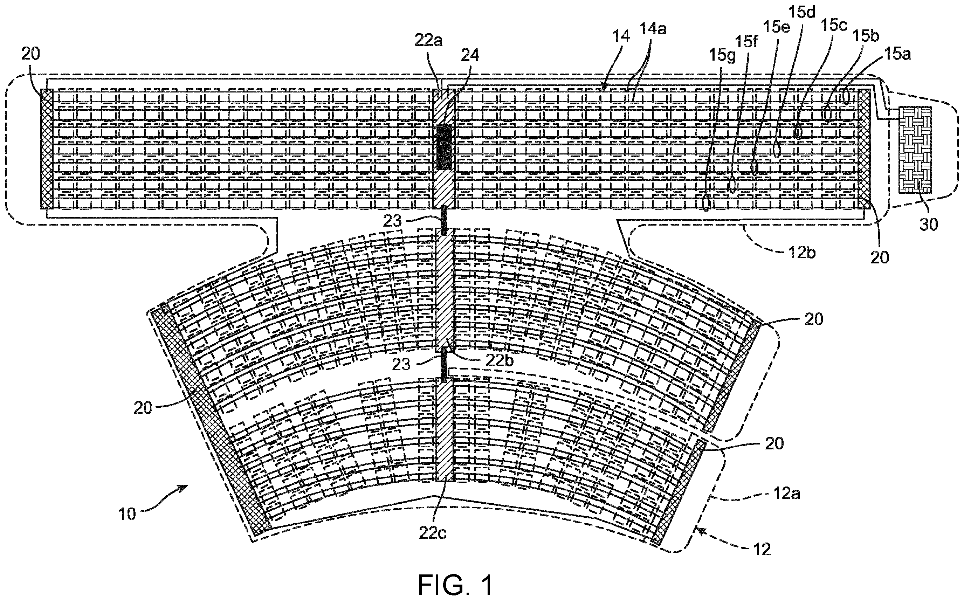

One embodiment of compression device 10 is shown in FIGS. 1-3. The device 10 in the illustrated embodiment is configured to be wrapped around the calf, but it is understood that the device can be modified as necessary for treatment of other extremities. The device 10 includes a textile or fabric body 12 having a lower segment 12a configured to fit around the foot of the user and an upper segment 12b configured to encircle the lower leg. The ends of each segment may include a hook and loop fastener arrangement to permit adjustable fit around the user's foot and calf. Other means for adjustably fastening the body segments about the user's body are contemplated, such as an array of hooks, eyelets, zipper, Velcro or similar fastening devices. The fastening devices may also be similar to the tightening mechanisms used in thoracic spinal bracing, backs packs and even shoes. It is further contemplated that the device may be a closed body that is integral around the circumference.

The fabric body 12 may be formed of a generally inelastic or only moderately "stretchable" material that is suited for contact with the skin of the user. The material of the fabric body may be a breathable material to reduce perspiration or may be a generally impermeable material to enhance heating of the body part under compression treatment. It is understood that the configuration of the body 12 shown in FIG. 1 can be modified according to the body part being treated. For instance, the fabric body 12 may be limited to the upper segment 12b to wrap the calf, thigh, bicep or forearm only. The body may also be configured to fit at the knee or elbow of the user. The fabric body may be provided with a "tacky" coating or strips on the surface facing the limb, with the "tacky" coating helping to hold the body against sliding along the user's limb, particularly if the user sweats beneath the fabric body.

In accordance with one feature of the present invention, the device is provided with a plurality of shape-changing elements that are operable to change shape in response to an external stimulus. This change of shape effectively reduces the circumference of the device encircling the user's limb, thereby applying pressure or a compressive force to the limb. In one embodiment, the shape-changing element is an element configured to change length, and more particularly to reduce its length in response to the stimulus. The shape-changing element is one or more wires formed of a "shape memory" material or alloy that shrinks when a current is applied to the wire, and that returns to its original "memory" configuration when the current is removed or changed. A wire array is provided that includes wires formed of a "memory" material that changes length upon application of an electrical signal and then returns to its original length when the signal is terminate. In a specific embodiment, the memory material can be a memory metal such as Nitinol or Dynalloy wire having a diameter of 0,02 cm (0.008 in). In one specific embodiment, the memory wires 14 are configured so that a current of 0.660 amp passing through each wires causes it to shrink sufficiently to exert a force of about 0,57 kg (1.26lbf) to 1,8 kg (4lbf) In other embodiments, the wire array may be formed of an auxetic material that expands when placed in and then returns to its initial thickness when the is removed.

As reflected in FIG. 1 each pair of wires 14a, corresponds to a single channel that can be individually actuated during a compression treatment. Each channel, or wire pair, 14a is connected to a microcontroller as described herein. In the illustrated embodiment, the upper segment 12b includes seven such channels 15a-15g. The lower segment 12a includes a wire array with seven channels and a wire array with six channels. Each row or channel of wires 14a in the wiring array 14 terminates at a negative anode or ground plane 20 at the opposite ends of each body segment 12a, 12b. Each channel, such as the channels 15a-15g, is electrically connected to a corresponding distribution circuit board 22a-22c. A flexible multiconductor cable 23 connects the distribution circuit boards between segments of the fabric body 12 so that the distribution circuit boards do not interfere with the ability of the device 10 to be wrapped snugly about the user's extremity.

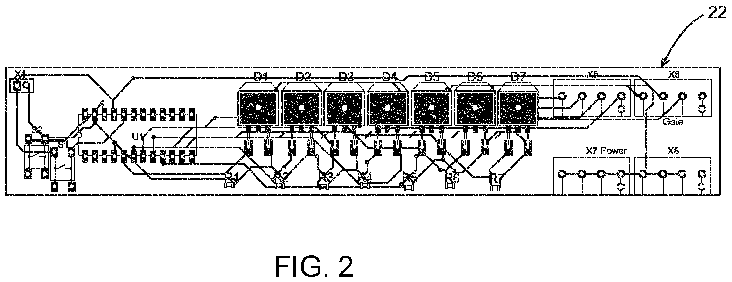

One of the distribution circuit boards 22a carries a microprocessor 24 that controls the sequence and magnitude of the current applied to the memory wires in each channel. As shown in FIG. 2, the distribution circuit boards 22 can include surface mount resistors and power mosfets electrically connected to the wire pairs of each channel. The microcontroller 24 is preferably not hard-wired to the circuit board 22a to permit replacement of one pre-programmed microcontroller with a differently programmed microcontroller. In one embodiment, a microcontroller may be preprogrammed with a particular compression sequence for a particular user and a particular device. For instance, the compression sequence may be an infinite or continuous rolling in which the device is successively compressed along the length of the user's limb similar to a peristaltic movement, a step-wise sequence in which the device is compressed and held for a period, or even a random sequence. Other compression protocols may be preprogrammed into other microcontrollers that can be selected by the user or physical therapist as desired.

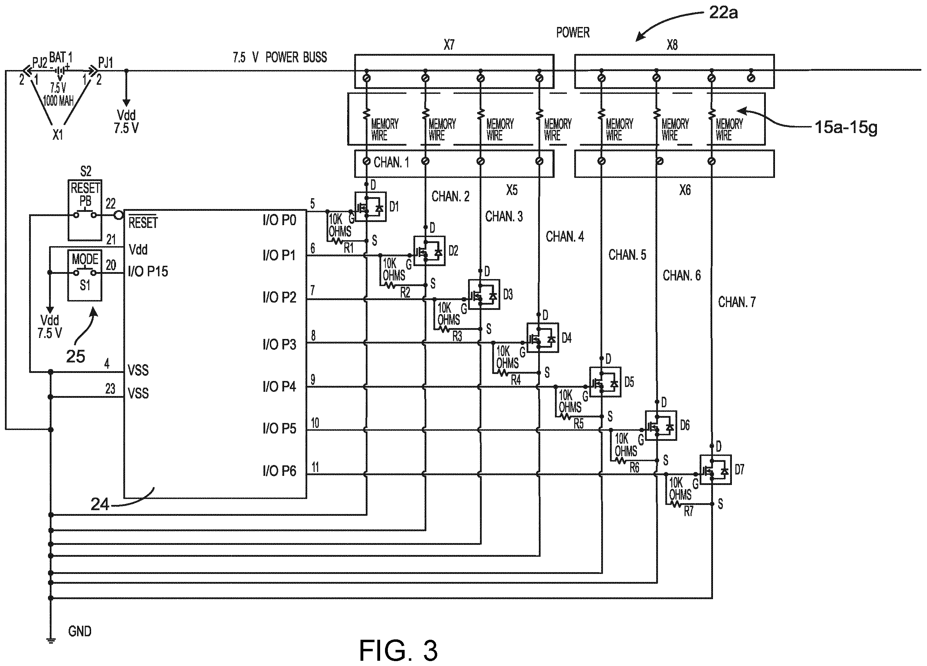

Details of the circuit board 22a and microcontroller 24 are shown in the circuit diagram of FIG. 3. The microcontroller may be a Parallax microcontroller Part No. BS2-IC, or a Bluetooth enabled Arduino microcontroller, for instance. The microcontroller is provided with a switch array 25 which includes a mode switch S1 and a reset switch S2. The switches are accessible by the user to operate the device 10. Alternatively, the switches may be integrated into a remote communication module capable of wireless communication from outside the compression device. The circuit board may thus incorporate a transmitter/receiver component coupled to the switches S1, S2, such as an RF, Bluetooth, wifi or Spec 802.11 device. The device 10 can be equipped with a USB type connection for charging the power supply 30 and for data download or upload. The microcontroller may thus include a memory for storing actuation data, and may further integrate with sensors on the circuit boards that can sense and "report" pressure and temperature, for instance. In one aspect, the microcontroller 24 is thus configured to communicate with a handheld device, such as an iPad, iPod, smart phone, or with another device equipped with wireless transmission/receiving capabilities, such as a PC or laptop computer. The remote device can serve to receive and record actuation data, and can act as a master controller for the micro-controller 24, whether to activate either of the two switches, or in a more advanced configuration to remotely configure or program the micro-controller. The remote device can be used to allow the user to tailor a particular compression protocol. In one aspect, the remote device can implement a software program that allows the user to obtain immediate feedback and provide instructions to the micro-controller 24 in before, during and after a compression sequence. The micro-controller 24 may provide compression readings to the remote device indicative of the amount of compression being applied by the device. In one embodiment, the compression readings can be based on the amount of actuation of the wire array or the current applied to the wire array. The user can use the compression readings during a compression sequence to determine a desirable, or tolerable, amount of compression to program future compression protocols.

A power supply 30 is provided that is connected to the distribution circuit boards 22a-22c and grounded to the negative anodes 20. In one embodiment, the power supply 30 is a 7.5 volt, 40AH lithium cell array contained with a pouch defined in the fabric body 12. The pouch may be configured to insulate the user from any heat build-up that might occur when the battery is powering the device 10. The power supply 30 is preferably a rechargeable battery that can be recharged through the remote link to the microcontroller described above.

The micro-controller 24 implements software for controlling the sequence and pattern of compression that will be followed through a treatment process. In one embodiment, the micro-controller is activated and controlled by a remote device, as described above. Additionally, the micro-controller can have basic user controls embedded in the device, such as a control panel affixed to the outside of one of the fabric segments 12a, 12b. The manner in which pressure is applied to the user's body depends upon the number and arrangement of the pads 16 and channels 15. The channels may be actuated from the lowermost channel 15g to the uppermost channel 15a, with successive channels being gradually deactivated, or expanded, and gradually activated, or contracted. Different activation patterns can be pre-programmed into the micro-controller or administered by the remote device as described above. When a channel is activated, the micro-controller 24 directs current to the specific channel which causes the memory wires 14a to contract or shrink, thereby reducing the effective diameter of the memory wires or elongated materials when wrapped around a limb. This reduction in diameter translates to an application of pressure in the same manner as the air-inflatable devices of the prior art. When the current is removed or changed, the "memory" feature of the wire allows it to return to its deactivated or neutral condition, thereby removing pressure. In addition, the limb or fascia acts as a spring to assist in returning the memory wire(s) to the neutral phase.

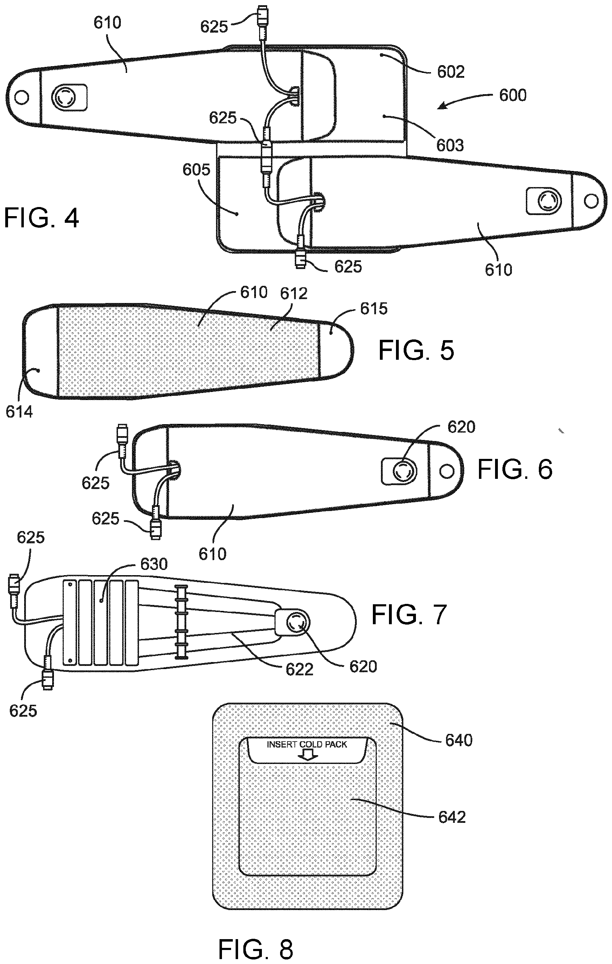

The compression devices disclosed herein may be provided in a multicomponent configuration. For example, as shown in FIGS. 4-8, a compression device 600 may be provided with a base panel 602 with an engagement surface 603, such as a hook-and-loop fastening surface. A pair of elongated panels 610 are provided, with each panel including a number of the plurality of ribs and at least two shape-changing wires, such as any of the rib and wire configurations described above. The elongated panels 610 are provided with an inward surface 612 configured to contact the user's skin, with the surface having a gripping texture to prevent slipping of the device in use. One end 614 of each panel is configured for attachment to the base panel 602, as depicted in FIG. 4. The opposite end 615 of each elongated panel is also configured for attachment to the base panel 602 when the device 600 is wrapped around the body of a user. The ends 614, 615 may be configured with a hook-and-loop fastening feature.

As shown in the partial cut-away view of FIG. 7, each elongated panel 610 includes an array of ribs 630 with SMA wires (not shown) that are connected to electrical couplings 625. The couplings 625 electrically connect the SMA wires of the two elongated panels 610 and can provide electrical connection to an external component, such as an external controller for controlling actuation of the SMA wires as described above.

In a further feature, the elongated panels 610 may be provided with a pre-tensioning element 620 configured to apply a tension across the panel when the device is engaged around a portion of the body of the user. The tensioning element 620 may be connected to one of the ribs 630 by cables 622 that are adapted to be placed in tension by the element 620. In one embodiment, the tensioning element 620 may be a rotating ratchet mechanism configured to wind the cables 622 to thereby place them in tension. The tensioning element 620 allows the user to apply some pre-tension to the device when worn. The pre-tension is maintained as the SMA wires are actuated. This feature thus allows the user to provide two stage compression, with the first stage provided by the tensioning element 620 and the second stage provided by the SMA wires. This two-stage tensioning thus allows for a greater maximum compression than with the SMA wires alone and thus accounts for the compression limits inherent with the SMA feature.

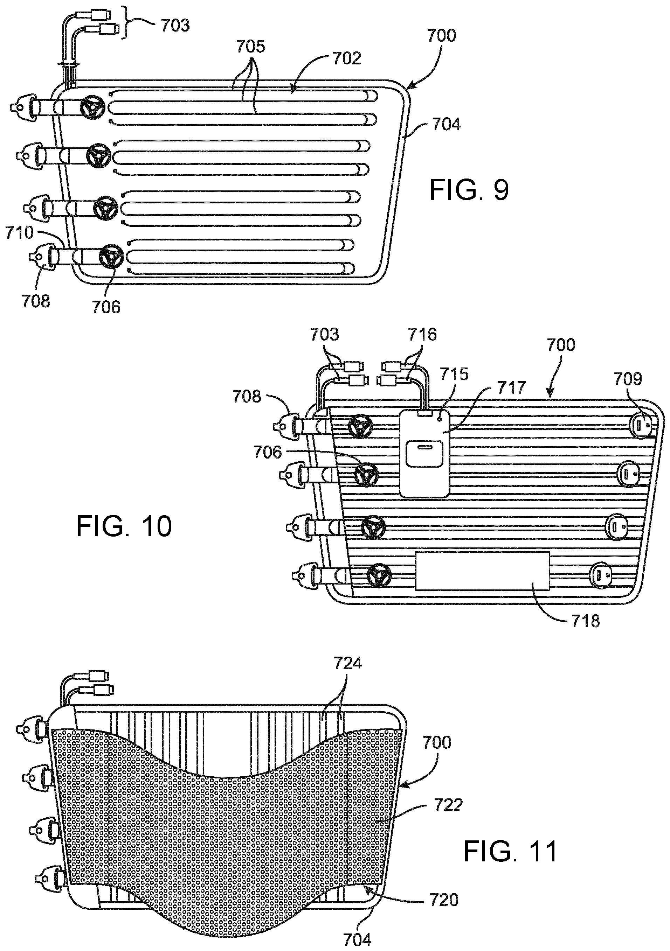

A compression device 700 shown in FIGS. 9-11 includes a compression assembly 702 mounted on a flexible panel 704 similar to the shape-changing compression assemblies described above. In one particular embodiment, the compression assembly 702 can include a plurality of SMA wires 705 extending along the length of the device. The compression assembly 702 includes cables 703 that are configured to mate with cables 716 of a control module 715 (FIG. 10) that may be held in a pocket 717 on the outer surface of the compression device 700. The control module 715 may be configured to provide a user interface for controlling the compression device or may simply incorporate a power supply, on/off button and wireless interface for communicating with a separate computer as described above.

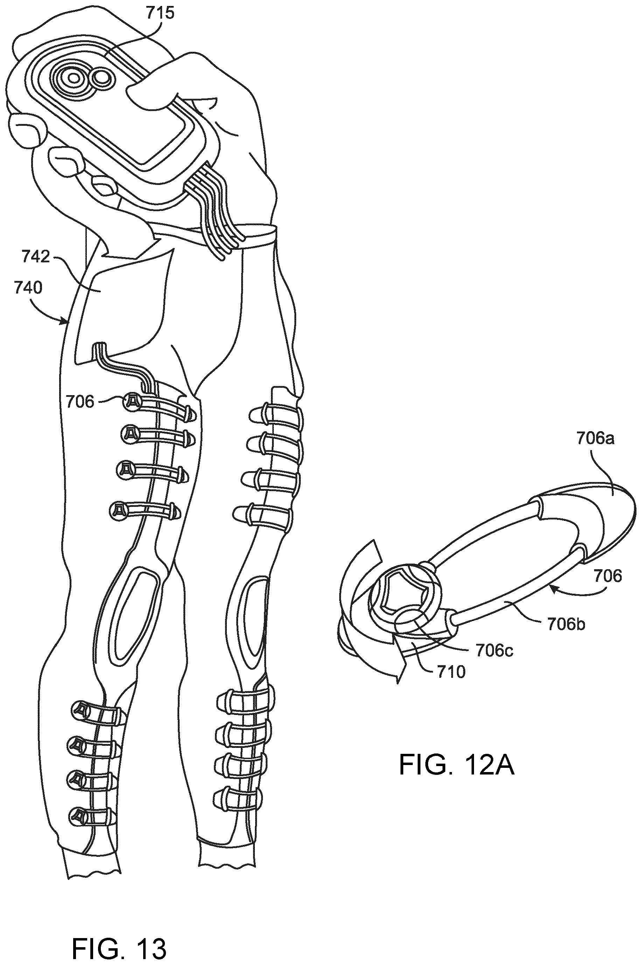

The panel 704 is thus configured to encircle a body portion, such as a limb, of a user in the manner described above. However, in a modification from the prior embodiments, the panel 704 includes quick-release connectors 708, 709 (FIGS. 9-10) that can be readily snapped together and released. One connector component 709 is affixed to a strap 710 that is integrated into a tensioning component 706. In one embodiment, the tensioning component may be a BOA closure system of BOA Technology, Inc. Details of the BOA closure system can be found in U.S. Patent No. 8,516,662. The BOA closure 706 as generally illustrated in FIG. 12A includes an anchor 706a that is affixed to the panel 704 and a tension dial component 706c that is affixed to the strap 710 connected to the quick-release connector component 708. The tension dial component shortens or lengthens the cable 706b as described in the '662 Patent to thereby adjust the tension in the cable 706b. The tensioning component thus provides a mechanism for the user to tighten the compression device 700 to a comfortable fit prior to activating the compression assembly 702. The tensioning component 706 provides the two-stage compression capability discussed above.

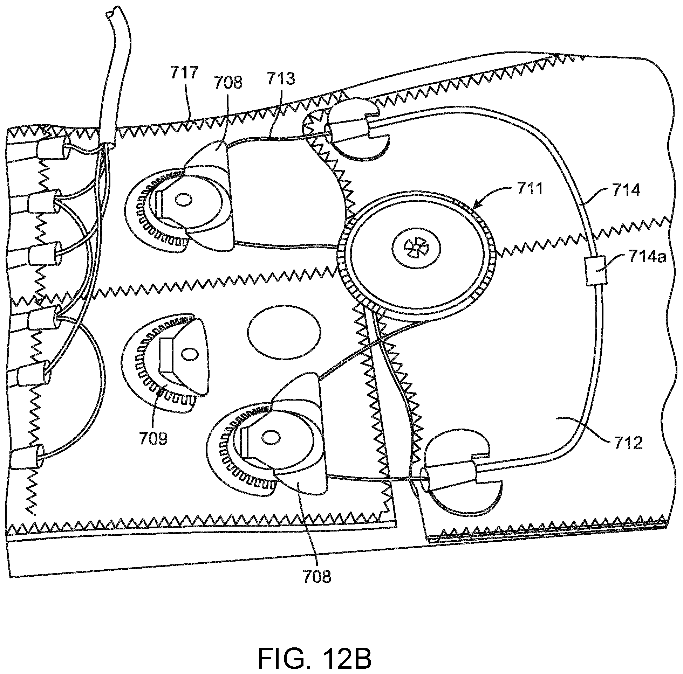

An alternative configuration using the BOA closure is depicted in FIG. 12B. In this configuration, the tension dial component 711 is affixed to one end 712 of a compression device with the cable 713 wound through the tension dial and through two quick-release connector components 708. The cable 713 further passes through a sheath 714 that is partially anchored to the compression device at location 714a. The two connector components 708 are configured to mate with corresponding components 709 that are affixed to an opposite end 717 of the compression device. It can be appreciated that the single tension dial component 711 can apply tension through two quick-release connector components 708/709 with this alternative configuration.

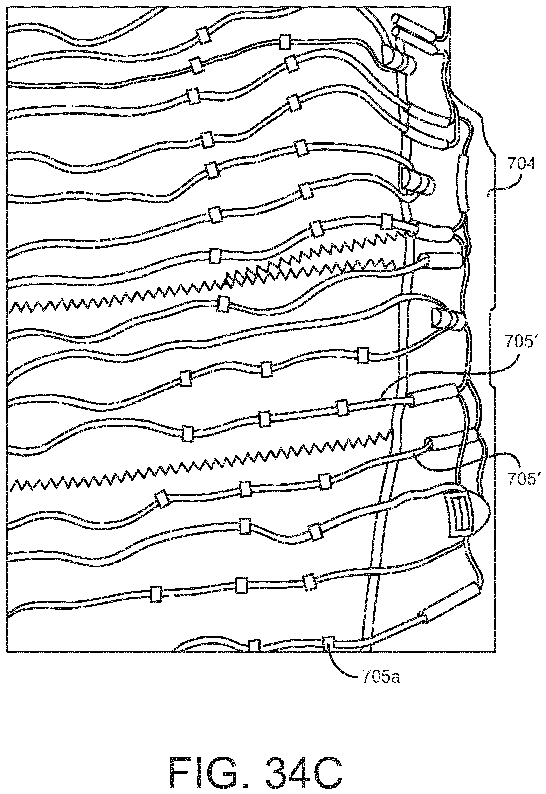

As a further alternative, the SMA wires themselves may be integrated into the BOA closure mechanism, rather than a separate cable, such as cable 713. In this alternative, the SMA wire, such as the SMA wires 705 shown in FIG. 9 pass through the quick-release components 708, 709 and through the tension dial component 711. In this approach, the SMA wires are necessarily longer than in the other embodiments. Preferably, the SMA wires are "wavy" in their unstressed configuration as illustrated by the wires 705' shown in FIG. 34C, rather than generally linear as shown in FIG. 9. The "wavy" wires 705' allow the slack in the wires to be taken up as the tension dial component is rotated. At the same time, the body of the compression device, such as panel 704, is stretched until the slack in the SMA wires is removed. The pre-tensioning thus applies tension to both the panel 704 and the SMA wires 705', with the wires being further tensioned during the operation of the compression device.

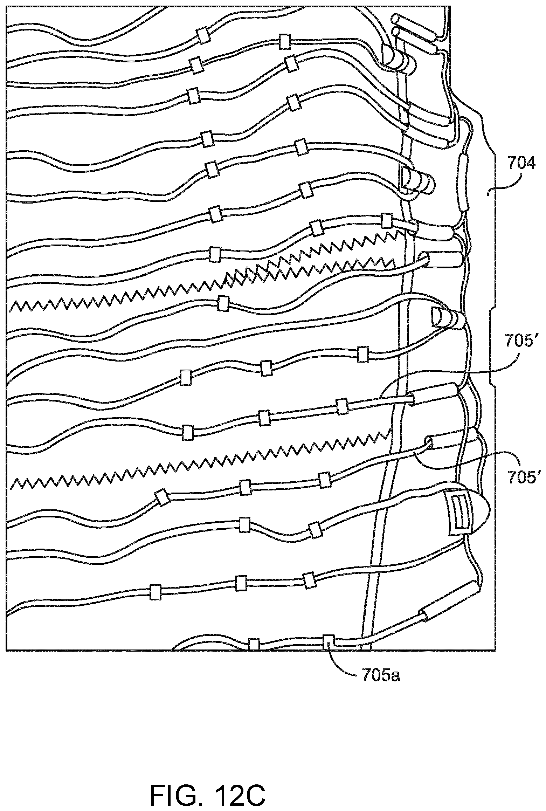

FIG. 12C illustrates a further feature, namely anchors 705a used to anchor the SMA wire 705' to the body or panel 704 of the compression device. The anchors hold the wires to the panel while allowing the wires to slide as the wires are tensioned and released. In one embodiment, the anchors 705a are loops fastened or sewn to the panel 704 are spaced locations along the length of the device. The anchors 705a are spaced far enough apart to allow the SMA wires to assume the "wavy" shape shown in FIG. 12C, but are also spaced close together enough to provide support the wires as they are activated.

Returning to FIG. 11, the inner face of the compression device 700 may include an athletic mesh inner liner 720. An elastic mesh sleeve 722 may be affixed to the ends of the panel 704 to form an opening through which the user inserts the body part being treated. The sleeve 722 is elastic enough to fit the body part, such as a leg, to help position the compression device 700 prior to fastening the quick-release connectors 708, 709. The inner liner 720 may include a series of sleeves 724 sized to receive heating or cooling strips to enhance the treatment with a form of thermal therapy.

As shown in FIG. 10, the outer face of the device 700 may include a pocket for storing the control module 715. In addition, a panel 718 may be provided on the outer face that allows the user to record information pertinent to the use of the compression device. In one embodiment, the panel 718 may be a dry-erase surface onto which the user may record a particular treatment protocol.

In the disclosed exemplary embodiments, the wires are arranged generally parallel to the extent of the device or fabric strap. In other words, the wires are arranged around parallel circumferences encircling the limb of the user. In alternative embodiments, the wires may be arranged at an angle relative to the circumference. With this configuration, the compression pressure applied by the device when actuated extends not only circumferentially around the limb but also includes a pressure component along the length of the limb. It is further contemplated that the wires may be coated or housed within a tube to help reduce the heat transmission as the wires are actuated. The coating or tubing may be formed of aramid, nylon, TEFLON or other similar low friction, and preferably low thermal conductivity, material.

In the disclosed exemplary embodiments, the compressive force is created by activation of a shape-changing element, whereby under a certain stimulus the element changes shape in a direction adapted to tighten the device about the user's limb. In some embodiments the shape-changing elements are single strand wires, such as memory metal wires, that are activated by flowing a current through and thus ohmically heating the wire. In other alternatives, the shape-changing elements may be braided wires that are activated by an ohmically heated wire passing through the interior of the braid.

In a further alternative, the shape-changing element may be an auxetic cable that changes aspect ratio rather than length. With this type of material, the thickness of the cable increases when the cable is activated, which translates into a radial pressure on the limb for a generally inelastic device. The auxetic cable is actuated by pulling the ends of the cable. A shape memory actuator may be utilized to provide the force to pull the ends of the auxetic cable. It is further contemplated that a micro-solenoid structure may be used to provide the pulling force. In this case, the micro-solenoid can be controlled to provide an oscillating pressure, such as by rapidly pulling and releasing the auxetic cable.

While the present disclosure is generally directed to human users, patients or athletes, the compression devices disclosed herein can be adapted to other animals. For instance, race horses often receive pre- and post-race treatments similar to those received by human athletes. Any of the compression devices disclosed herein may be sized and configured to encircle any part of the leg of a horse. Similar modifications can be made for treatment of other animals as well.

Moreover, the SMA wires described herein may be actuated by the application of an electrical current, such as a typical shape memory alloy. The SMA wires will thus generate heat as the current flows through the wires. This heat may be part of the treatment regimen using the compression devices of the present disclosure. Alternatively, the SMA wires may be thermally isolated to avoid heat transfer to the patient.

The compression devices disclosed herein may be used in Enhanced External Counterpulsation Therapy (EECP) as non-surgical treatment for angina. One or more devices may be wrapped around the lower extremities of a patient to physically squeeze blood back to the heart on each heart beat. The controller of the devices may be linked to or incorporate a pulse sensor that evaluates the heart beat of the patient and then times the activation of the SMA wires to provide compression at an appropriate time in the heart beat cycle. The controller can then operate according to one of the protocols described above in which sections of the compression devices are actuated in sequence to help push blood back to the heart.

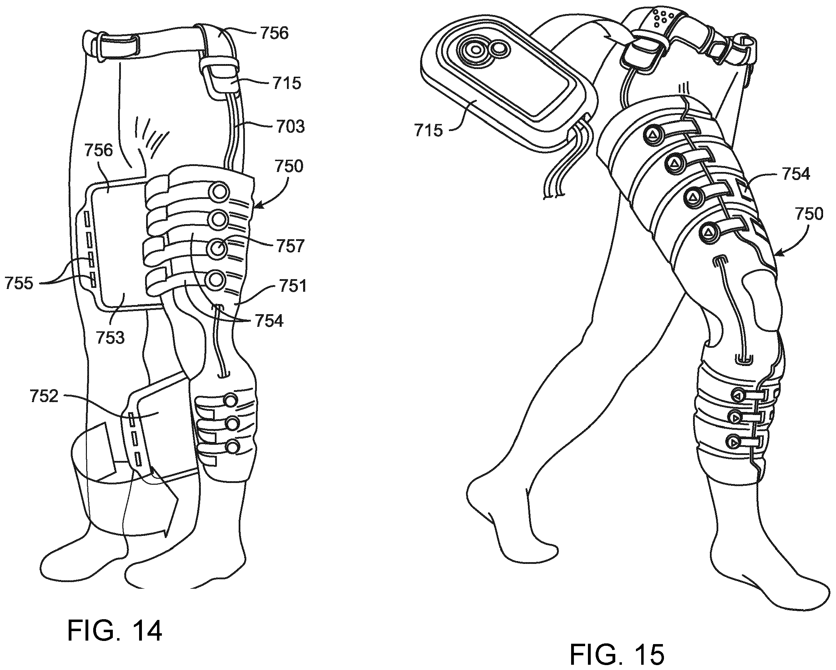

The compression devices disclosed herein may be used to treat nearly all muscles of the body. As shown in FIG. 13, the compression device can be integrated into a wearable full lower body compression device 740 with compression applied at each leg 706. A pocket 742 can be incorporated to store the control module 715 during the compression treatment. The compression device 750 shown in FIGS. 14-15 is configured to be wearable on a user's leg outside existing clothing. In this embodiment, individual segments 752, 753 are wrapped around the thigh and calf, respectively, and are connected by a segment 751. Each segment is initially held in position by straps 754 that are threaded through slots 755 in the panel 756. As shown in FIG. 14, the straps 754 may be integrated into a tensioning device 757 as described above. Each strap 754 may have hook-and-loop surfaces to either fasten back onto itself or fasten against the outer surface of the device 750, as shown in FIG. 15.

A further feature is the addition of a liner 753 that incorporates a cooling material, such as a cooling gel. The liner 753 may be pre-cooled, such as in the refrigerator, and then added to the device 750, such as by removable attachment or fitting within a pre-formed pocket, similar to the pouch 640 described above. Alternatively, the liner 753 may be a "nubbed" panel, namely a panel that includes relatively hard plastic nubs that bear against the skin. It can be appreciate that the liner 753 may be incorporated into all of the compression devices disclosed herein, whether configured with a cooling gel or as a "nubbed" panel or any other configuration adapted for therapeutic treatment. The control module 715 is connected to the compression assembly associated with the device 750 by a cable 703 and may be carried by a belt strap 756 configured to wrap around a waist belt.

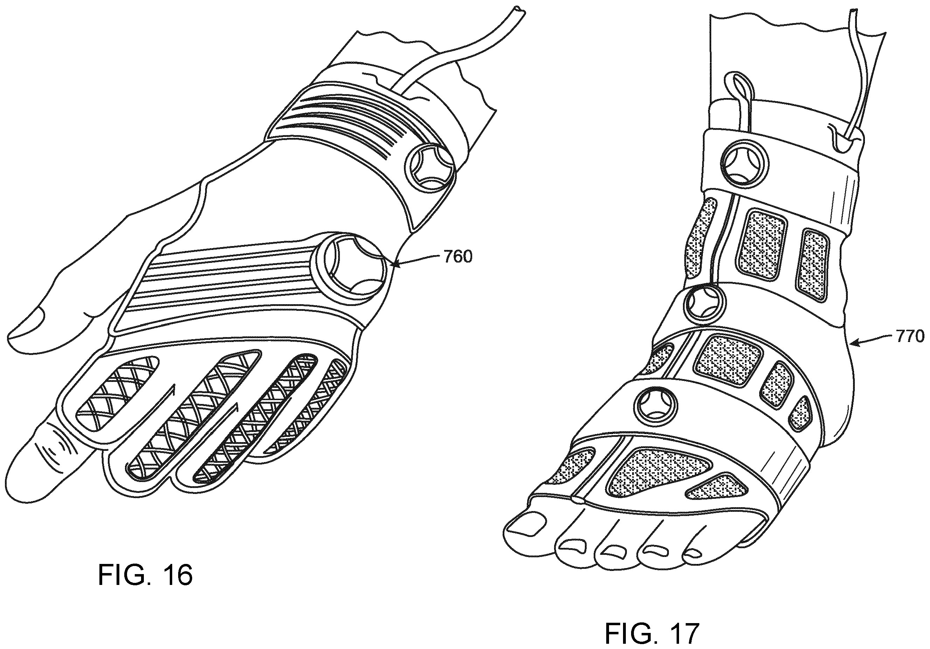

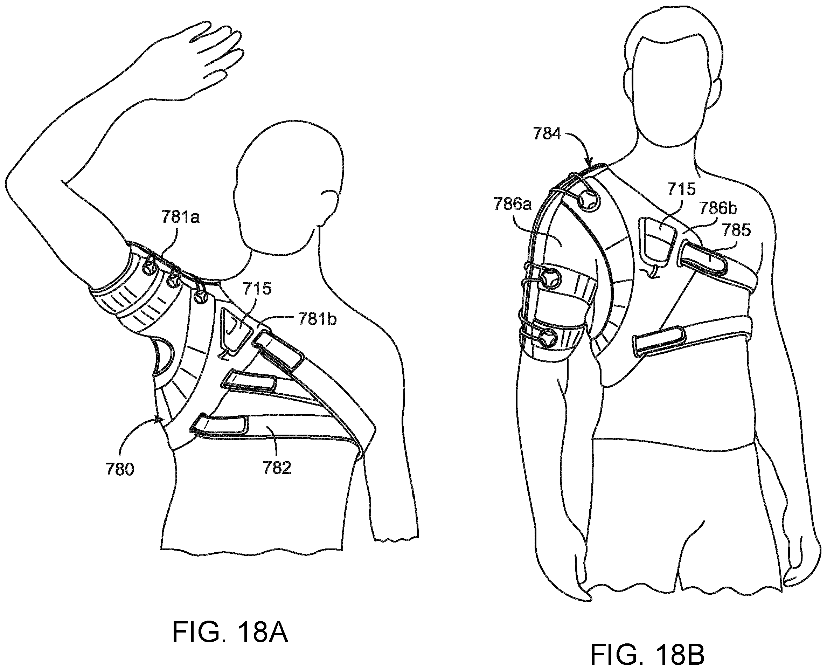

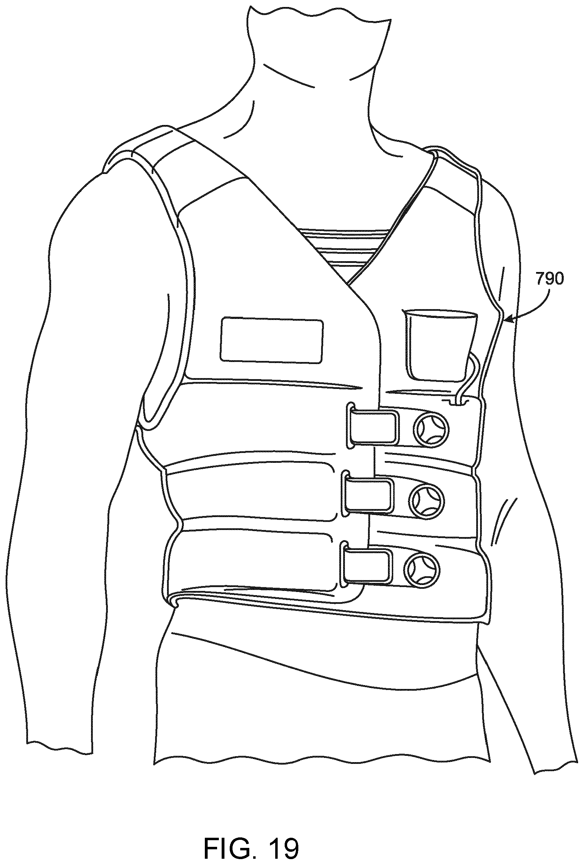

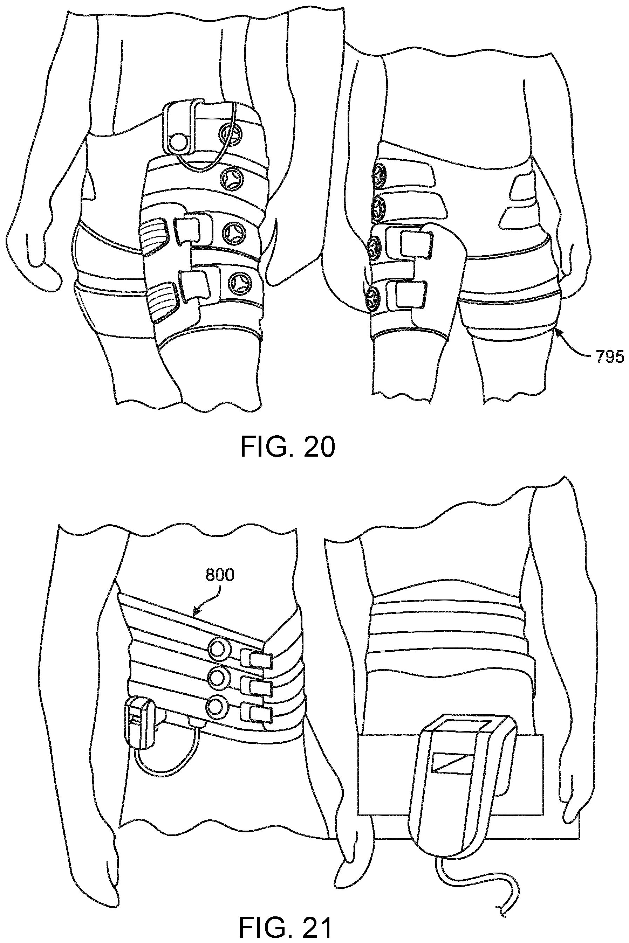

The compression devices described herein may be further modified to fit other parts of the body, such as the compression device 760 configured to be wrapped around the hand, and the device 770 configured as an ankle wrap, as shown in FIGS. 16, 17, respectively. A shoulder arrangement is shown in FIGS. 18A, 18B. The shoulder compression devices 780, 784 are configured to wrap around the upper arm and shoulder. The devices 780, 784 include a portion 781a, 786a that wraps around the upper arm and a portion 781b, 786b extends across the chest and upper back of the user. The devices are held in position by chest straps 782, 785, respectively, that extend from the portions 781b, 786b and wrap around the chest and back of the user. The devices may be provided with pockets for carrying the control module 715. A compression device 790 shown in FIG. 19can be configured as a vest to be wrapped around the chest or torso of the user, particularly to apply compression to the back muscles. The compression device 795 shown in FIG. 20 is configured to be worn like workout shorts to provide compression to the hip, IT band and gluteus muscles of the wearer. FIG. 21 shows a lumbar compression device 800 specifically configured to apply compression to the lower back. A calf compression device 810 shown in FIG. 22 includes multiple panels 811-814 extending from a base panel 816. Two of the panels 811, 812 are arranged to wrap around the lower calf of the user, while the panels 813, 814 are configured to be worn around the upper portion of the calf. The panels include quick-release connector components 818, 819 and a tension dial 820 similar to the connector and pre-tensioning components described above. A shield panel 822 may also be provided to overlap and cover the ends of the panels 811-814 when they are connected to the base panel around the calf of a user.

The compression device may be integrated into a shoe to provide compression or massage for the user's foot. Thus, as shown in FIGS. 23A-23C, a shoe 900 with a sole 901 and an upper 902 includes a compression device 910 disposed between the sole 901 and the shoe insert (not shown). The compression device 910 includes SMA wires 915 that extend along the sole and along the inside of the shoe upper 902. A cap 920 is provided between the user's foot and the SMA wires 910 so that the wires are disposed between the shoe upper and the cap. The cap 920 is sufficiently rigid to protect the top of the user's foot from direct pressure from the SMA wires. The cap also helps distribute the compression force as the SMA wires are activated. The shoe insert protects the bottom of the user's foot. The compression device 910 can thus operate to apply a massage or compression protocol to the user's foot.

An alternative device 930 is shown in FIGS. 24A, 24B implemented within a shoe. The sole 932 of the shoe serves as an anchor for separate compression devices 935, 937 and houses the power and on-board control circuitry 940. The compression devices 935, 937 include corresponding SMA wires 936, 938 that are fed through loops 942 mounted to the shoe upper 934 in a generally sinusoidal pattern, as shown in FIGS. 24A, 24B. When the respective devices 935, 937 are activated the apply a compressive force C (FIG. 24B) at the center of the foot and at the Achilles tendon portion of the shoe to tighten the shoe on the user's foot. The power and control circuitry 940 may be activated by downward pressure by the user's heel on a switch embedded within the circuitry, or can be activated by an external switch. The external switch may be integrated into the sole 932 or the upper 934 of the shoe or may be part of a wireless component so that the device 930 can be selectively actuated by a smart phone. The SMA wires 936, 938 can be integrated into the material of the sole 932 and upper 934 to help insulate the SMA wire so that it does not lose heat too quickly, thereby allowing the SMA wire to hold its level of contraction with less power input to the wire.

In a further modification, the compression device at the center of the shoe can be modified as shown in FIG. 24C. In particular, the compression device 935' can incorporate individual wires 936 that extend from the shoelace eyelets L and are embedded within the sole 932. The wires 936 are preferably placed within sheaths that are embedded within the sole 932 to allow the wires to contract within the sole of the shoe. The wires may also be embedded within the material of the shoe upper 934, also desirably within a sheath.

The compression devices disclosed herein incorporate a controller that controls the actuation of the SMA wires to apply the compression-release protocols described herein. The controllers for the compression devices may incorporate a microcontroller, such as microcontrollers 24 or 66 described above, that controls the duty cycle of the current applied to each of the SMA wires of the particular device. The microcontrollers may incorporate pulse-width modulation techniques to control the actuation time of the SMA wires to protect from overheating the wires. By way of example, the microcontrollers are configured to apply current to an actuated SMA wire long enough to achieve a 2-15% reduction in length or shrinkage of the wire over a predetermined time period. In specific examples, a voltage of 5-30V and a current of 0.2-4 amps is applied for a duration of 1.5-3.5 seconds. The duty cycles can vary between 35-100%. The PWM frequencies can be in the range of 2-10kHz. This controlled actuation applies the desired compression for a time period that provides a desirable compression sequence for the user without generating too much heat. The sequence in which successive SMA wires are actuated can be used to provide sufficient time for a wire to cool down before being actuated again. Since the actuation properties of the SMA wire are a function of the length and diameter of the wires, the microcontroller can be provided with MOSFET switches corresponding to certain predetermined wire lengths/diameters.

The present disclosure contemplates compression devices that are worn on the body of a person. This application of SMA wires involves certain challenges to providing uniform, controllable contraction of the device while also preventing electrical shock and over-heating of the user, and while further doing so in an energy efficient manner. In accordance with the devices disclosed herein, it is contemplated that a power source in the range of 5-50 watts can be required to produce effective results for large scale compression. This power requirement can be satisfied by a 12 volt power supply, whether by the use of on-board high-energy batteries, such as lithium polymer batteries, or by the use of an electrical adapter/transformer connected to a separate electrical source.

A further complexity is providing a useful combination of force and stroke with the an SMA wire device. A typical SMA wire, such as a Nitinol wire, can contract about 4% of its length when heated. Thus, simply winding a single SMA wire around a body part, such as a thigh, does not provide sufficient compression for most purposes. For instance, it has been found that an 8-10% decrease in the diameter of a large muscle, such as the thigh, is necessary for an optimum "feel" and effectiveness. Moreover, the compression force needs to be distributed over a larger area in order to avoid the single wire "garroting" effect. Greater force distribution militates in favor of a larger diameter SMA wire, which can provide greater compressive forces but at the cost of greater energy input requirements.

Another factor in the complexity of an SMA-based wearable compression device is the speed of the contraction. In some applications of the present compression device, a therapeutic compression can be achieved with a rolling two second compression wave. The resultant energy expenditure of this compression wave leads to waste heat that must be removed or the device can worm up to levels that are uncomfortable to the user. Thus, the present device contemplates a combination of energy management, thermal insulation and activation cycle time to optimize the speed of contraction and its effects on the user.

The devices disclosed herein incorporate an SMA wire that is coated or encapsulated in an elastomeric material. As shown in the diagram of FIG. 25, a wire arrangement 1000 includes an SMA wire 1001, which can be similar to the SMA wires discussed above, that is shrouded within an elastomeric sheath 1003. The sheath 1003 is formed of a silicone or polyurethane material. In one embodiment, the sheath 1003 may be formed directly on or coated onto the SMA wire 1001. The elastic nature of the sheath material allows the sheath to expand and contract with the SMA wire. The sheath may have a thickness calibrated to provide a larger diameter for the wire arrangement 1000 than the SMA wire 1001 itself. This larger overall diameter helps distribute the force produced by the wire arrangement when the SMA wire is contracted. Alternatively, a gap 1004 may exist between the sheath 1003 and the SMA wire 1001 so that the wire can slide independent of the sheath. The gap 1004 provides an additional level of insulation beyond the thickness of the sheath itself. On the other hand, the gap 1004 is not necessary for actuation of the wire arrangement and can lead to bunching of the sheath when the compression device is activated.

The material of the sheath 1003 is adapted to have a thermal conductivity sufficient to help the SMA wire 1001 maintain its temperature upon contraction with only limited additional energy input required to maintain the same level of compression. The thermal mass and specific heat capacity of the sheath can be calibrated by changing the thickness of the coating material applied to the SMA wire. According to the invention, the sheath material is doped with a thermally conductive or thermally insulative material to adjust the overall conductivity of the sheath. For instance, the sheath 1003 may be infused with air bubbles or may incorporate glass beads or diamond dust. The sheath 1003 may selectively incorporate insulative components immediately adjacent the body 1010 of the underlying fabric that contacts the user's skin, and may selectively incorporate thermally conductive components on the outer surface. The SMA wires are, in effect, resistive heaters so the temperature increase of the SMA wires is known if the voltage pulse durations to the wires is known. The thermal conductivity, diffusivity and thermal mass of the sheath regulates the thermal energy leaving the wire arrangement 1000 as well as the heat experienced by the user wearing the compression device.

Returning to FIG. 25, a further benefit of the sheath 1003 is depicted. In particular, the sheath can facilitate attachment of the wire arrangement 1000 to the body of the compression device. In one embodiment, the sheath 1003 may be affixed to an elastic fabric 1006 of the compression device. The fabric 1006 may be formed of a material capable of spreading the compression forces that are generated by contraction of the SMA wire. Thus, the fabric may be a bamboo or nylon material that can be curved to fit the shape of the user's body. In a further aspect the SMA wire 1001' of compression device 1010' may be formed in an initial sinusoidal configuration, as depicted in FIG. 26A. The sinusoidal configuration allows the SMA wire 1001' to act like a spring. The wire can then readily stretch or straighten as the fabric 1006 stretches. Thus, as the device is mounted on the body of the user, the fabric 1006 of the device stretches and the SMA wires 1001" become substantially straightened, as depicted in FIG. 26B. This straightening of the SMA wires serves to apply a pre-tension to the wires. When the SMA wires 1001" are energized they attempt to return to their original sinusoidal shape, thereby applying compression to the user's body.

In a further embodiment, the SMA wire 1001 and silicone sheath 1003 can be incorporated into an arrangement that incorporates a second sheath 1012, as shown in FIGS. 27A, 27B. As shown in FIG. 27A, the silicone sheath 1003 is formed into a U-shape, with one end anchored to an anchor piece 1019 for connection to an adjustable closure 1018, which can be similar to the closure 706 shown in FIG. 12A. The SMA wire 1001 is anchored to an underlying fabric layer, such as fabric 1006 in FIG. 25, by anchors 1002. The silicone sheath 1003 is affixed to a less pliant tubing, such as nylon tubing 1012, by fittings 1014. The fittings 1014 may be in the form of shrink tubing that is heat shrunk around both tubings 1003, 1012 to secure fix the tubing together. The fittings 1014 may be fastened to a separate anchor strip 1016 that is fastened to the underlying fabric. The same fitting can be used to affix the nylon tubing to the SMA wire anchors 1002, as shown in FIG. 27B. The nylon tubing has a low friction inner surface but is otherwise generally less pliant than the silicone tubing 1003. This combination of tubing focuses the stroke of the compression device to the silicone tubing 1003, thereby providing a well-controlled region of compression.

The voltage duty cycle can be regulated to regulate not only the energy entering the system but also the energy leaving the system. Reducing the thickness of the sheath 1003 increase the thermal energy expelled from the wire arrangements 1000, which thermal energy manifests in an increase in temperature of the compression device to the user, which may be desirable. Alternatively, increasing the thickness of the sheath 1003 decreases the thermal energy exiting the wire arrangement so that the compression device no longer performs like a heating pad. However, limiting the thermal energy leaving the wire arrangement means that more heat is retained by the SMA wire, which in turn means that less additional energy is required to keep the SMA wire arrangement at its level of contraction.

In some cases it is desirable to cycle the wire arrangement between the contracted condition (i.e., Austenitic phase of the SMA wire) and the relaxed condition (i.e., Martinsitic phase of the SMA wire). In this case a balance must be maintained between the input energy and the thermal energy that is either stored/retained by the sheath and the energy that is dissipated through the sheath. In this case, shorter input pulses of higher voltages may be preferred, with the understanding that the higher input voltages produces faster actuation speeds for the device. Tight control can be achieved by monitoring the current in the SMA wire 1001. For example, a Nitinol wire of 20 cm (50 inch) length and 0,006 cm (0.015 inch) diameter in a silicone sheath pressed against the body can require 21 volts to heat the wire to full phase change in about 2.5 seconds. The initial current pulse is about 1.7 amps and then peaks to about 2 amps after the phase change. If there is a positive heat transfer from the wire arrangement to the body a steady state condition can be achieved in which the energy applied to the wire arrangement equals the energy exiting. When the current can be increased no further, the wire temperature can be maintained at just above the Austenitic state nearly indefinitely. On the other hand, if the heat transfer is negative, meaning that heat is transferred from the person's body to the wire arrangement, the wire temperature can rise quickly once the SMA wire reaches its Austenitic phase. It is at this point that the controller can turn off the voltage applied to the wire arrangement to regulate both the contraction of the wire arrangement and the heat transfer condition between the user and the wire arrangement. In addition, at this point the voltage may continue to be applied to the wire arrangement in order to achieve a particular heating effect during a compression cycle, being careful to avoid overheating the SMA wire. In a typical case the SMA wire can be damaged if its temperature exceeds 130°C.

The device controller monitors the current flow through the SMA wires, and may in particular monitors the change in slope of the current pulse. If the slope falls below a threshold the electrical current is shut off to the SMA wire. In one example, that threshold is when the slope drops below 0.1 amp per second increase. The current can be measured by Hall-effect sensors or similar means, and the slope can be calculated in real-time by the controller and stored throughout each actuation cycle for continuous evaluation. As a fail-safe, a maximum voltage pulse on-time can be established, such as 2.5 seconds for a given actuation cycle. It is contemplated that the actuation time for a compression device according to the present disclosure may require a longer cycle time when the device is first turned on because the SMA wires are at ambient temperature well below the phase transition temperature. It is further contemplated that with each successive actuation cycle the actuation time will decrease because the SMA wire has already reached the transition temperature and the ability of the sheath to shed thermal energy gradually decreases as the user's body temperature inside the compression device increases. Under these conditions it is then necessary to shorten the voltage pulses to prevent the SMA wire from over-heating. Under certain conditions, extended "rest" for the compression device and wire arrangements maybe necessary to allow the SMA to cool below the transition temperature and below the Martinsitic finish temperature. Thus, in some cases this rest period can be 60-90 seconds.

In one method for controlling the current flow through the SMA wires, the length of the wires can be continuously sampled and then the current shut off to the wires when they reach their fully transformed length. Thus, in one aspect, a time domain reflectometer (TDR) or a frequency domain reflectometer (FDR) can be used to measure the initial length of the SMA wire and then to continuously sample the wire length. The TDR or FDR can be configured to signal the device controller to turn the power off when the wire shrinks to its full transformation length. In another approach, a mechanical switch may be provided in lieu of the TDR or FDR. Thus, as shown in FIG. 28, the SMA wire 1020 may be anchored to an end connector 1024 and slidably disposed within a sheath 1022. Electrical contacts 1025 are mounted to the end connector 1024, and contacts 1026 are mounted to the end of the sheath 1022, with wires 1027 communicating with the device controller. When the SMA wire 1020 shrinks to its desired length, the contracting wire draws the end connector 1024 toward the sheath 1022 until the electrical contacts 1025, 1026 meet. When the contacts meet, the wire circuit 1027 is closed to send a signal to the device controller which responds accordingly to stop current flow to the SMA wire 1020.

In a further embodiment, a compression device 1050 combines the active compression features of the SMA wire devices described above with an electrical stimulation device. Thus, as shown in FIG. 29, a compression device 1050 can include a base 1052 configured to be worn on the user, such as in the manner of any of the compression devices described herein. The device is provided with SMA wires 1054 that are powered and controlled by a device controller 1065, again in any of the manners described herein. The base 1052 includes a stimulation region 1060 on which is mounted a plurality of electrical stimulation devices 1062. In one embodiment, the devices are TENS- transcutaneous electrical nerve stimulation - units. The TENS units 1062 are connected to the device controller 1065 which is configured to activate and control the TENS units according to a desired protocol, such as by controlling pulse width, frequency and intensity of the electrical signal emanating from the devices. The compression device 1050 can position at least one SMA wire 1054 over each TENS unit 1062 to help press and maintain the TENS unit in direct contact with the user's skin, which is necessary for the TENS units to operate. Since the SMA wire electrical requirements and duty cycle may differ from the TENS unit duty cycle and electrical requirements, the TENS units 1062 are separately wired to the device controller 1065.

The device controller 1065 can be configured to modulate the SMA wires and TENS units according to a desired protocol. For instance, in one protocol the SMA wires 1054 are activated to provide compression for a fixed period, say four seconds, and then while the SMA wires are relaxed the TENS units 1062 are activated for another four seconds, followed by four seconds of rest. This cycle can be repeated as long as desired, and may be accompanied by the addition of heat to the compression and/or TENS activation parts of the cycle.

In operation, each SMA wire 502a, 502b is separately controllable, as described above. When one wire, such as wire 502a, is activated, the wire contracts in length so that the ribs essentially slide relative to the wire 502a to be drawn together at the end 501c of each rib. A similar action occurs when the second wire 502b is actuated. Since the wires are not constrained within the ribs 501, a single wire can be used to contract each end of the compression device. The two wires can be actuated in a predetermined sequence to achieve a pulsing compression as desired.

The compression devices disclosed herein may be provided in a multicomponent configuration. For example, as shown in FIGS. 26-30, a compression device 600 may be provided with a base panel 602 with an engagement surface 603, such as a hook-and-loop fastening surface. A pair of elongated panels 610 are provided, with each panel including a number of the plurality of ribs and at least two shape-changing wires, such as any of the rib and wire configurations described above. The elongated panels 610 are provided with an inward surface 612 configured to contact the user's skin, with the surface having a gripping texture to prevent slipping of the device in use. One end 614 of each panel is configured for attachment to the base panel 602, as depicted in FIG. 26. The opposite end 615 of each elongated panel is also configured for attachment to the base panel 602 when the device 600 is wrapped around the body of a user. The ends 614, 615 may be configured with a hook-and-loop fastening feature.

As shown in the partial cut-away view of FIG. 29, each elongated panel 610 includes an array of ribs 630 with SMA wires (not shown) that are connected to electrical couplings 625. The couplings 625 electrically connect the SMA wires of the two elongated panels 610 and can provide electrical connection to an external component, such as an external controller for controlling actuation of the SMA wires as described above.

In a further feature, the elongated panels 610 may be provided with a pre-tensioning element 620 configured to apply a tension across the panel when the device is engaged around a portion of the body of the user. The tensioning element 620 may be connected to one of the ribs 630 by cables 622 that are adapted to be placed in tension by the element 620. In one embodiment, the tensioning element 620 may be a rotating ratchet mechanism configured to wind the cables 622 to thereby place them in tension. The tensioning element 620 allows the user to apply some pre-tension to the device when worn. The pre-tension is maintained as the SMA wires are actuated. This feature thus allows the user to provide two stage compression, with the first stage provided by the tensioning element 620 and the second stage provided by the SMA wires. This two-stage tensioning thus allows for a greater maximum compression than with the SMA wires alone and thus accounts for the compression limits inherent with the SMA feature.

In an additional feature, the compression device 600 may be provided with a removable pouch 640 shown in FIG. 30. The pouch 640 may be removably mounted to the base panel 602, such as at a location 605. The pouch 640 may be configured to receive a cooling or heating element 642 as desired by the user.

A compression device 700 shown in FIGS. 31-33 includes a compression assembly 702 mounted on a flexible panel 704 similar to the shape-changing compression assemblies described above. In one particular embodiment, the compression assembly 702 can include a plurality of SMA wires 705 extending along the length of the device The compression assembly 702 includes cables 703 that are configured to mate with cables 716 of a control module 715 (FIG. 32) that may be held in a pocket 717 on the outer surface of the compression device 700. The control module 715 may be configured to provide a user interface for controlling the compression device or may simply incorporate a power supply, on/off button and wireless interface for communicating with a separate computer as described above.

The panel 704 is thus configured to encircle a body portion, such as a limb, of a user in the manner described above. However, in a modification from the prior embodiments, the panel 704 includes quick-release connectors 708, 709 (FIGS. 31-32) that can be readily snapped together and released. One connector component 709 is affixed to a strap 710 that is integrated into a tensioning component 706. In one embodiment, the tensioning component may be a BOA closure system of BOA Technology, Inc. Details of the BOA closure system can be found in U.S. Patent No. 8,516,662 (the '662 Patent), which issued in August 27, 2013, the disclosure of which is incorporated herein by reference. The BOA closure 706 as generally illustrated in FIG. 34A includes an anchor 706a that is affixed to the panel 704 and a tension dial component 706c that is affixed to the strap 710 connected to the quick-release connector component 708. The tension dial component shortens or lengthens the cable 706b as described in the '662 Patent to thereby adjust the tension in the cable 706b. The tensioning component thus provides a mechanism for the user to tighten the compression device 700 to a comfortable fit prior to activating the compression assembly 702. The tensioning component 706 provides the two-stage compression capability discussed above.

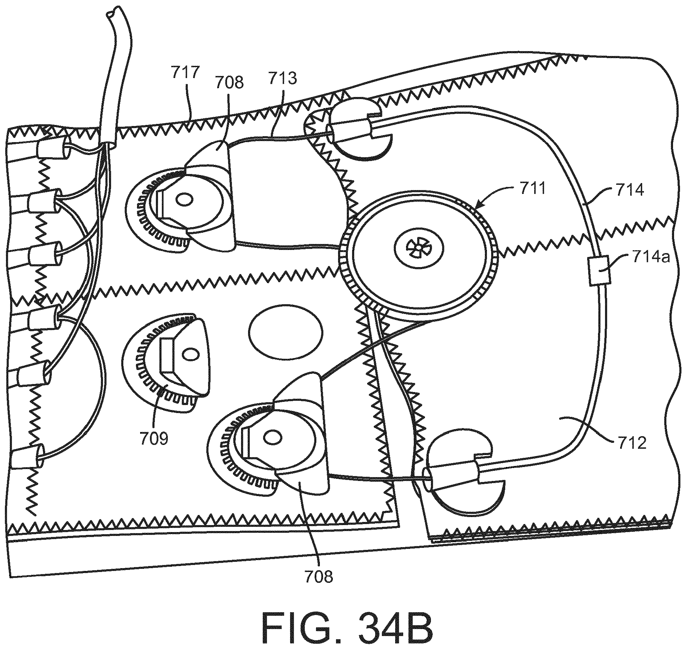

An alternative configuration using the BOA closure is depicted in FIG. 34B. In this configuration, the tension dial component 711 is affixed to one end 712 of a compression device with the cable 713 wound through the tension dial and through two quick-release connector components 708. The cable 713 further passes through a sheath 714 that is partially anchored to the compression device at location 714a. The two connector components 708 are configured to mate with corresponding components 709 that are affixed to an opposite end 717 of the compression device. It can be appreciated that the single tension dial component 711 can apply tension through two quick-release connector components 708/709 with this alternative configuration.

As a further alternative, the SMA wires themselves may be integrated into the BOA closure mechanism, rather than a separate cable, such as cable 713. In this alternative, the SMA wire, such as the SMA wires 705 shown in FIG. 31, the wires 110a-c described in connection with FIG. 10, or the SMA wires shown in the embodiments of FIGS. 8 and 9. Thus, rather than a separate cable passing through the quick-release components 708, 709 and through the tension dial component 711, the SMA wires, such as wires 705 can pass through those components. In this approach, the SMA wires are necessarily longer than in the other embodiments. Preferably, the SMA wires are "wavy" in their unstressed configuration as illustrated by the wires 705' shown in FIG. 34C, rather than generally linear as shown in FIG. 31. The "wavy" wires 705' allow the slack in the wires to be taken up as the tension dial component is rotated. At the same time, the body of the compression device, such as panel 704, is stretched until the slack in the SMA wires is removed. The pre-tensioning thus applies tension to both the panel 704 and the SMA wires 705', with the wires being further tensioned during the operation of the compression device.

FIG. 34C illustrates a further feature, namely anchors 705a used to anchor the SMA wire 705' to the body or panel 704 of the compression device. The anchors hold the wires to the panel while allowing the wires to slide as the wires are tensioned and released. In one embodiment, the anchors 705a are loops fastened or sewn to the panel 704 are spaced locations along the length of the device. The anchors 705a are spaced far enough apart to allow the SMA wires to assume the "wavy" shape shown in FIG. 34C, but are also spaced close together enough to provide support the wires as they are activated.

Returning to FIG. 33, the inner face of the compression device 700 may include an athletic mesh inner liner 720. An elastic mesh sleeve 722 may be affixed to the ends of the panel 704 to form an opening through which the user inserts the body part being treated. The sleeve 722 is elastic enough to fit the body part, such as a leg, to help position the compression device 700 prior to fastening the quick-release connectors 708, 709. The inner liner 720 may include a series of sleeves 724 sized to receive heating or cooling strips to enhance the treatment with a form of thermal therapy.

As shown in FIG. 32, the outer face of the device 700 may include a pocket for storing the control module 715. In addition, a panel 718 may be provided on the outer face that allows the user to record information pertinent to the use of the compression device. In one embodiment, the panel 718 may be a dry-erase surface onto which the user may record a particular treatment protocol.

In the disclosed exemplary embodiments, the wires are arranged generally parallel to the extent of the device or fabric strap. In other words, the wires are arranged around parallel circumferences encircling the limb of the user. In alternative embodiments, the wires may be arranged at an angle relative to the circumference. With this configuration, the compression pressure applied by the device when actuated extends not only circumferentially around the limb but also includes a pressure component along the length of the limb. It is further contemplated that the wires may be coated or housed within a tube to help reduce the heat transmission as the wires are actuated. The coating or tubing may be formed of aramid, nylon, TEFLON or other similar low friction, and preferably low thermal conductivity, material.

In the disclosed exemplary embodiments, the compressive force is created by activation of a shape-changing element, whereby under a certain stimulus the element changes shape in a direction adapted to tighten the device about the user's limb. In some embodiments the shape-changing elements are single strand wires, such as memory metal wires, that are activated by flowing a current through and thus ohmically heating the wire. In other alternatives, the shape-changing elements may be braided wires that are activated by an ohmically heated wire passing through the interior of the braid.

In a further alternative, the shape-changing element may be an auxetic cable that changes aspect ratio rather than length. With this type of material, the thickness of the cable increases when the cable is activated, which translates into a radial pressure on the limb for a generally inelastic device. The auxetic cable is actuated by pulling the ends of the cable. A shape memory actuator may be utilized to provide the force to pull the ends of the auxetic cable. It is further contemplated that a micro-solenoid structure may be used to provide the pulling force. In this case, the micro-solenoid can be controlled to provide an oscillating pressure, such as by rapidly pulling and releasing the auxetic cable.

While the present disclosure is generally directed to human users, patients or athletes, the compression devices disclosed herein can be adapted to other animals. For instance, race horses often receive pre- and post-race treatments similar to those received by human athletes. Any of the compression devices disclosed herein may be sized and configured to encircle any part of the leg of a horse. Similar modifications can be made for treatment of other animals as well.

Moreover, the SMA wires described herein may be actuated by the application of an electrical current, such as a typical shape memory alloy. The SMA wires will thus generate heat as the current flows through the wires. This heat may be part of the treatment regiment using the compression devices of the present disclosure. Alternatively, the SMA wires may be thermally isolated to avoid heat transfer to the patient.

As a further alternative, the compression devices or devices disclosed herein can be configured to apply focused pressure on a portion of the body without encircling the body. For instance, a device such as the device 400 may include a limited number of ribs, for example the three ribs shown in FIG. 18. The ribs may be removably adhered to the skin of a patient, such as across or along the lower back. Actuation of the SMA wires cause the space between ribs to successively reduce and expand as the wires contract and return to their neutral length. This action in effect kneads the skin as the device contract and expands. This approach allows the compression devices disclosed herein to be used as a training aid in which the device is worn by an athlete and is controlled to apply a compression force in response to an improper motion. For instance, the device can be adhered the triceps region of the arm of a golfer to apply a compressive force to the back of the arm in response to the golfer's elbow not being straight during a swing. Sensors associated with the device can determine the attitude of the golfer's arm and the relative position of the forearm and upper arm. The slight compressive force applied by the device can cause the golfer to tighten the triceps to thereby straighten the arm. Practice with the compression device generates a muscle memory so that the golfer learns to keep the elbow straight during a swing. The device can be used at any joint of the body to promote proper form for any type of repetitive sports motion, whether kicking a soccer ball, shooting a basketball or executing a butterfly swimming stroke.

The compression devices disclosed herein may be used in Enhanced External Counterpulsation Therapy (EECP) as non-surgical treatment for angina. One or more devices may be wrapped around the lower extremities of a patient to physically squeeze blood back to the heart on each heart beat. The controller of the devices may be linked to or incorporate a pulse sensor that evaluates the heart beat of the patient and then times the activation of the SMA wires to provide compression at an appropriate time in the heart beat cycle. The controller can then operate according to one of the protocols described above in which sections of the compression devices are actuated in sequence to help push blood back to the heart.

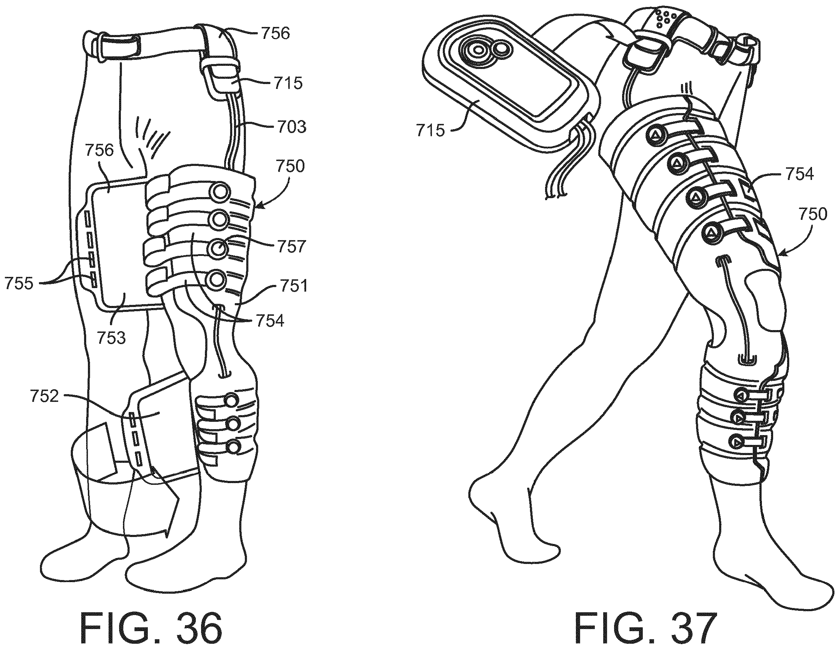

The compression devices disclosed herein may be used to treat nearly all muscles of the body. As shown in FIG. 35, the compression device can be integrated into a wearable full lower body compression device 740 with compression applied at each leg 706. A pocket 742 can be incorporated to store the control module 715 during the compression treatment. The compression device 750 shown in FIGS. 36-37 is configured to be wearable on a user's leg outside existing clothing. In this embodiment, individual segments 752, 753 are wrapped around the thigh and calf, respectively, and are connected by a segment 751. Each segment is initially held in position by straps 754 that are threaded through slots 755 in the panel 756. As shown in FIG. 36, the straps 754 may be integrated into a tensioning device 757 as described above. Each strap 754 may have hook-and-loop surfaces to either fasten back onto itself or fasten against the outer surface of the device 750, as shown in FIG. 37.

A further feature is the addition of a liner 753 that incorporates a cooling material, such as a cooling gel. The liner 753 may be pre-cooled, such as in the refrigerator, and then added to the device 750, such as by removable attachment or fitting within a pre-formed pocket, similar to the pouch 640 described above. Alternatively, the liner 753 may be a "nubbed" panel, namely a panel that includes relatively hard plastic nubs that bear against the skin. It can be appreciate that the liner 753 may be incorporated into all of the compression devices disclosed herein, whether configured with a cooling gel or as a "nubbed" panel or any other configuration adapted for therapeutic treatment. The control module 715 is connected to the compression assembly associated with the device 750 by a cable 703 and may be carried by a belt strap 756 configured to wrap around a waist belt.

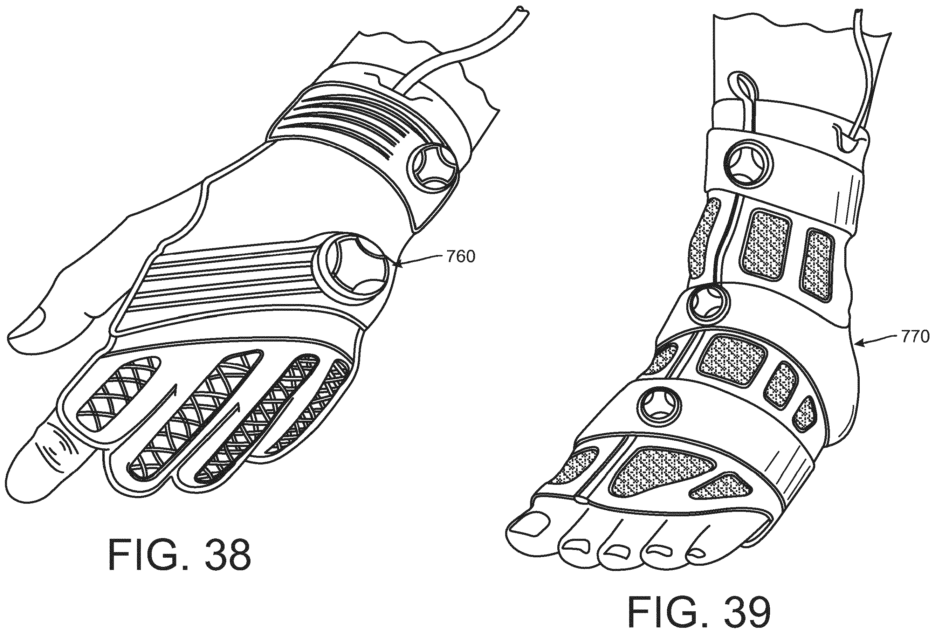

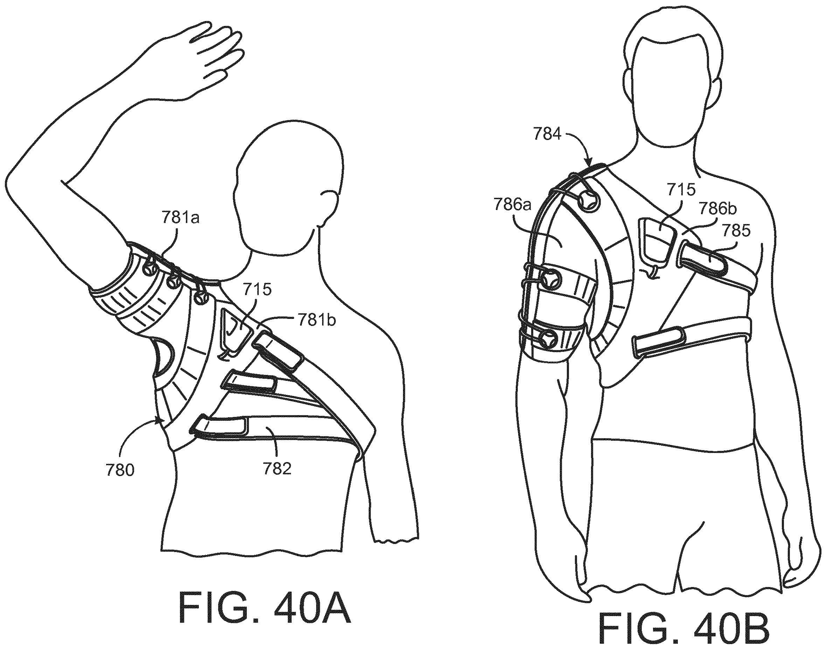

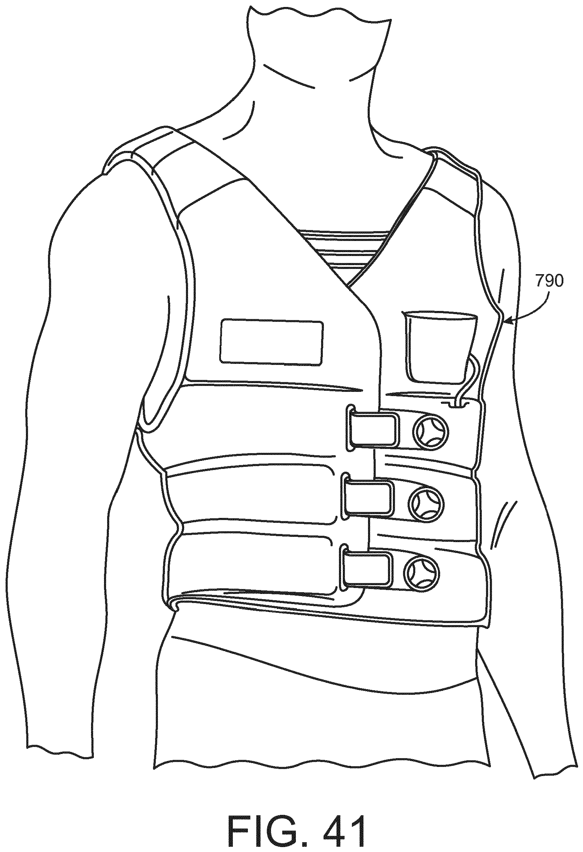

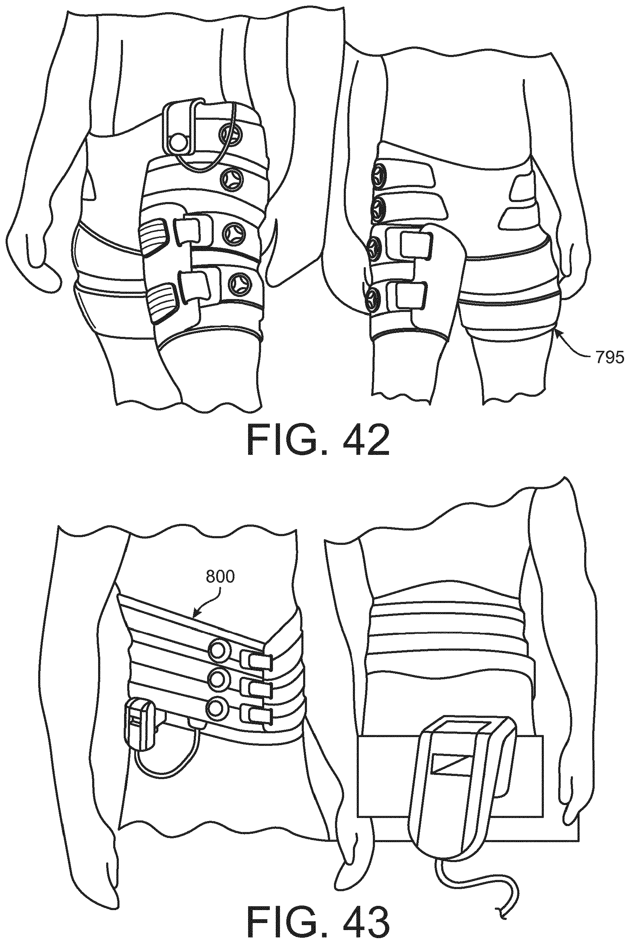

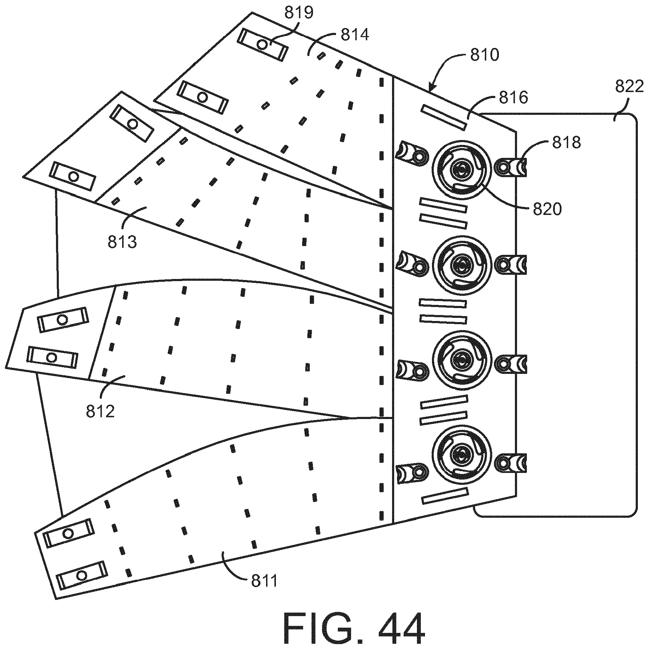

The compression devices described herein may be further modified to fit other parts of the body, such as the compression device 760 configured to be wrapped around the hand, and the device 770 configured as an ankle wrap, as shown in FIGS. 38, 39, respectively. A shoulder arrangement is shown in FIGS. 40a, 40b. The shoulder compression devices 780, 784 are configured to wrap around the upper arm and shoulder. The devices 780, 784 include a portion 781a, 786a that wraps around the upper arm and a portion 781b, 786b extends across the chest and upper back of the user. The devices are held in position by chest straps 782, 785, respectively, that extend from the portions 781b, 786b and wrap around the chest and back of the user. The devices may be provided with pockets for carrying the control module 715. A compression device 790 shown in FIG. 41can be configured as a vest to be wrapped around the chest or torso of the user, particularly to apply compression to the back muscles. The compression device 795 shown in FIG. 42 is configured to be worn like workout shorts to provide compression to the hip, IT band and gluteus muscles of the wearer. FIG. 43 shows a lumbar compression device 800 specifically configured to apply compression to the lower back. A calf compression device 810 shown in FIG. 44 includes multiple panels 811-814 extending from a base panel 816. Two of the panels 811, 812 are arranged to wrap around the lower calf of the user, while the panels 813, 814 are configured to be worn around the upper portion of the calf. The panels include quick-release connector components 818, 819 and a tension dial 820 similar to the connector and pre-tensioning components described above. A shield panel 822 may also be provided to overlap and cover the ends of the panels 811-814 when they are connected to the base panel around the calf of a user.

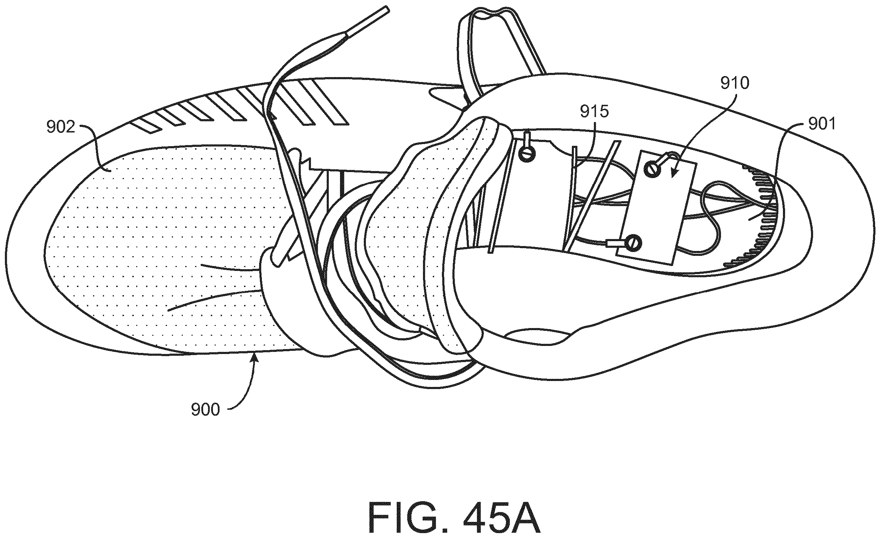

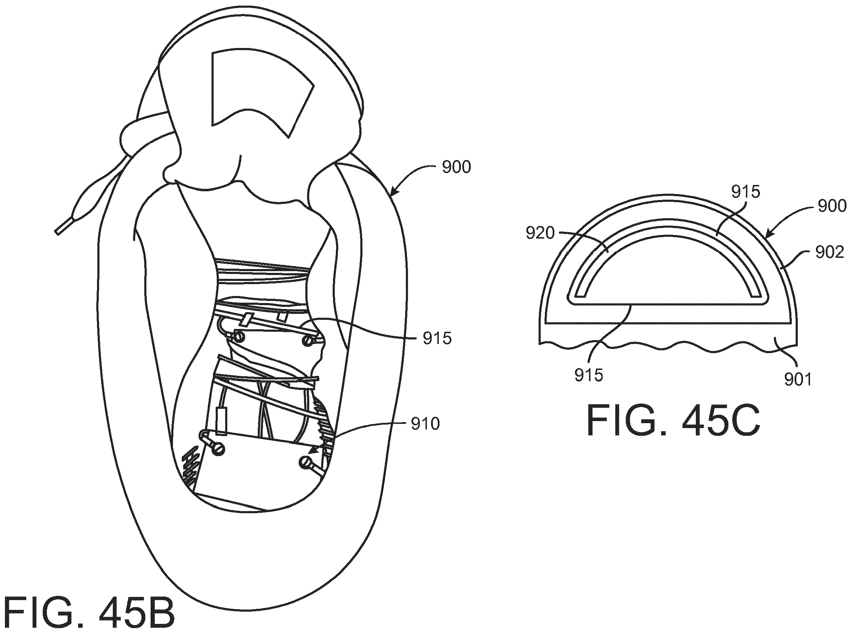

The compression device may be integrated into a shoe to provide compression or massage for the user's foot. Thus, as shown in FIGS. 45A-C, a show 900 with a sole 901 and an upper 902 includes a compression device 910 disposed between the sole 901 and the shoe insert (not shown). The compression device 910 includes SMA wires 915 that extend along the sole and along the inside of the shoe upper 902. A cap 920 is provided between the user's foot and the SMA wires 910 so that the wires are disposed between the shoe upper and the cap. The cap 920 is sufficiently rigid to protect the top of the user's foot from direct pressure from the SMA wires. The cap also helps distribute the compression force as the SMA wires are activated. The shoe insert protects the bottom of the user's foot. The compression device 910 can thus operate to apply a massage or compression protocol to the user's foot.

An alternative device 930 is shown in FIGS. 46A, 46B implemented within a shoe. The sole 932 of the shoe serves as an anchor for separate compression devices 935, 937 and houses the power and on-board control circuitry 940. The compression devices 935, 937 include corresponding SMA wires 936, 938 that are fed through loops 942 mounted to the shoe upper 934 in a generally sinusoidal pattern, as shown in FIGS. 46A, 46B. When the respective devices 935, 937 are activated the apply a compressive force C (FIG. 46B) at the center of the foot and at the Achilles tendon portion of the shoe to tighten the shoe on the user's foot. The power and control circuitry 940 may be activated by downward pressure by the user's heel on a switch embedded within the circuitry, or can be activated by an external switch. The external switch may be integrated into the sole 932 or the upper 934 of the shoe or may be part of a wireless component so that the device 930 can be selectively actuated by a smart phone. The SMA wires 936, 938 can be integrated into the material of the sole 932 and upper 934 to help insulate the SMA wire so that it does not lose heat too quickly, thereby allowing the SMA wire to hold its level of contraction with less power input to the wire.

In a further modification, the compression device at the center of the shoe can be modified as shown in FIG. 46C. In particular, the compression device 935' can incorporate individual wires 936 that extend from the shoelace eyelets L and are embedded within the sole 932. The wires 936 are preferably placed within sheaths that are embedded within the sole 932 to allow the wires to contract within the sole of the shoe. The wires may also be embedded within the material of the shoe upper 934, also desirably within a sheath.

The compression devices disclosed herein incorporate a controller that controls the actuation of the SMA wires to apply the compression-release protocols described herein. The controllers for the compression devices may incorporate a microcontroller, such as microcontrollers 24 or 66 described above, that controls the duty cycle of the current applied to each of the SMA wires of the particular device. The microcontrollers may incorporate pulse-width modulation techniques to control the actuation time of the SMA wires to protect from overheating the wires. By way of example, the microcontrollers are configured to apply current to an actuated SMA wire long enough to achieve a 2-15% reduction in length or shrinkage of the wire over a predetermined time period. In specific examples, a voltage of 5-30V and a current of 0.2-4 amps is applied for a duration of 1.5-3.5 seconds. The duty cycles can vary between 35-100%. The PWM frequencies can be in the range of 2-10kHz. This controlled actuation applies the desired compression for a time period that provides a desirable compression sequence for the user without generating too much heat. The sequence in which successive SMA wires are actuated can be used to provide sufficient time for a wire to cool down before being actuated again. Since the actuation properties of the SMA wire are a function of the length and diameter of the wires, the microcontroller can be provided with MOSFET switches corresponding to certain predetermined wire lengths/diameters.

The present disclosure contemplates compression devices that are worn on the body of a person. This application of SMA wires involves certain challenges to providing uniform, controllable contraction of the device while also preventing electrical shock and over-heating of the user, and while further doing so in an energy efficient manner. In accordance with the devices disclosed herein, it is contemplated that a power source in the range of 5-50 watts can be required to produce effective results for large scale compression. This power requirement can be satisfied by a 12 volt power supply, whether by the use of on-board high-energy batteries, such as lithium polymer batteries, or by the use of an electrical adapter/transformer connected to a separate electrical source.

A further complexity is providing a useful combination of force and stroke with the an SMA wire device. A typical SMA wire, such as a Nitinol wire, can contract about 4% of its length when heated. Thus, simply winding a single SMA wire around a body part, such as a thigh, does not provide sufficient compression for most purposes. For instance, it has been found that an 8-10% decrease in the diameter of a large muscle, such as the thigh, is necessary for an optimum "feel" and effectiveness. Moreover, the compression force needs to be distributed over a larger area in order to avoid the single wire "garroting" effect. Greater force distribution militates in favor of a larger diameter SMA wire, which can provide greater compressive forces but at the cost of greater energy input requirements.

Another factor in the complexity of an SMA-based wearable compression device is the speed of the contraction. In some applications of the present compression device, a therapeutic compression can be achieved with a rolling two second compression wave. The resultant energy expenditure of this compression wave leads to waste heat that must be removed or the device can worm up to levels that are uncomfortable to the user. Thus, the present device contemplates a combination of energy management, thermal insulation and activation cycle time to optimize the speed of contraction and its effects on the user.

Since a single SMA wire is generally inadequate to provide sufficient compression for meaningful utility, some sort of stroke amplification is required. The devices disclosed above provide one mechanism for stroke amplification, such as the devices shown in FIGS. 9-25. Further approaches are disclosed below.

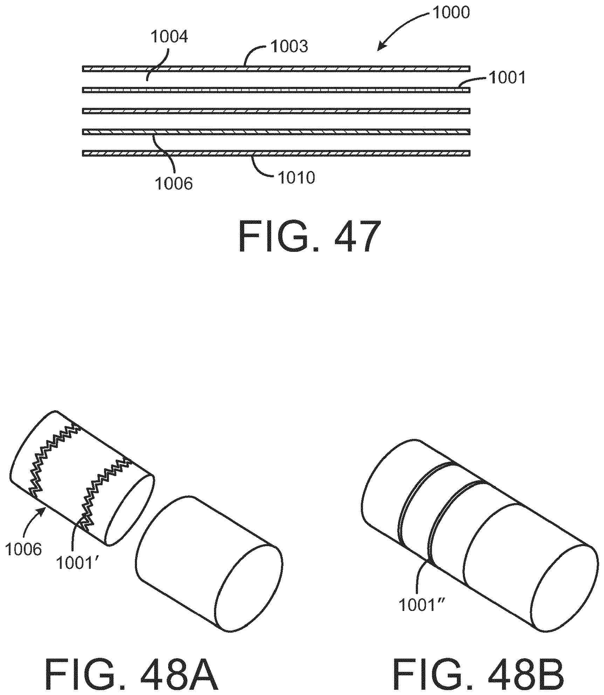

The devices disclosed herein can incorporate an SMA wire that is coated or encapsulated in an elastomeric material. Thus, in one embodiment shown in the diagram of FIG. 47, a wire arrangement 1000 includes an SMA wire 1001, which can be similar to the SMA wires discussed above, that is shrouded within an elastomeric sheath 1003. In one embodiment, the sheath 1003 can be formed of a silicone or polyurethane material. In one embodiment, the sheath 1003 may be formed directly on or coated onto the SMA wire 1001. The elastic nature of the sheath material allows the sheath to expand and contract with the SMA wire. The sheath may have a thickness calibrated to provide a larger diameter for the wire arrangement 1000 than the SMA wire 1001 itself. This larger overall diameter helps distribute the force produced by the wire arrangement when the SMA wire is contracted. Alternatively, a gap 1004 may exist between the sheath 1003 and the SMA wire 1001 so that the wire can slide independent of the sheath. The gap 1004 provides an additional level of insulation beyond the thickness of the sheath itself. On the other hand, the gap 1004 is not necessary for actuation of the wire arrangement and can lead to bunching of the sheath when the compression device is activated.

The material of the sheath 1003 is adapted to have a thermal conductivity sufficient to help the SMA wire 1001 maintain its temperature upon contraction with only limited additional energy input required to maintain the same level of compression. The thermal mass and specific heat capacity of the sheath can be calibrated by changing the thickness of the coating material applied to the SMA wire. In addition, the basic sheath material can be doped with a thermally conductive or thermally insulative material to adjust the overall conductivity of the sheath. For instance, the sheath 1003 may be infused with air bubbles or may incorporate glass beads or diamond dust. The sheath 1003 may selectively incorporate insulative components immediately adjacent the body 1010 of the underlying fabric that contacts the user's skin, and may selectively incorporate thermally conductive components on the outer surface. The SMA wires are, in effect, resistive heaters so the temperature increase of the SMA wires is known if the voltage pulse durations to the wires is known. The thermal conductivity, diffusivity and thermal mass of the sheath regulates the thermal energy leaving the wire arrangement 1000 as well as the heat experienced by the user wearing the compression device.

Returning to FIG. 47, a further benefit of the sheath 1003 is depicted. In particular, the sheath can facilitate attachment of the wire arrangement 1000 to the body of the compression device. In one embodiment, the sheath 1003 may be affixed to an elastic fabric 1006 of the compression device. The fabric 1006 may be formed of a material capable of spreading the compression forces that are generated by contraction of the SMA wire. Thus, the fabric may be a bamboo or nylon material that can be curved to fit the shape of the user's body. In a further aspect the SMA wire 1001' of compression device 1010' may be formed in an initial sinusoidal configuration, as depicted in FIG. 48Aa. The sinusoidal configuration allows the SMA wire 1001' to act like a spring. The wire can then readily stretch or straighten as the fabric 1006 stretches. Thus, as the device is mounted on the body of the user, the fabric 1006 of the device stretches and the SMA wires 1001" become substantially straightened, as depicted in FIG. 48B. This straightening of the SMA wires serves to apply a pre-tension to the wires. When the SMA wires 1001" are energized they attempt to return to their original sinusoidal shape, thereby applying compression to the user's body.

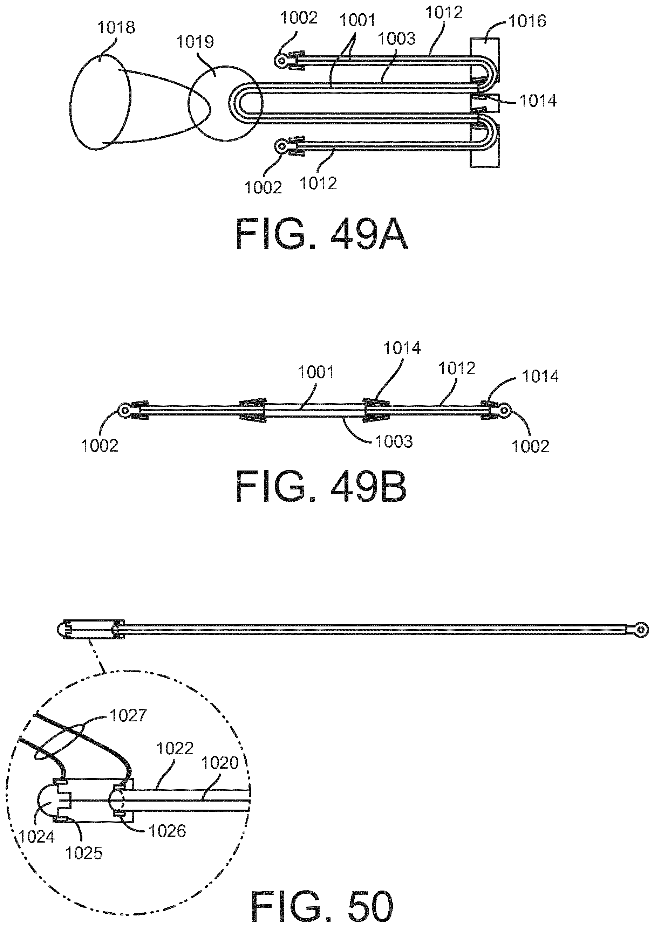

In a further embodiment, the SMA wire 1001 and silicone sheath 1003 can be incorporated into an arrangement that incorporates a second sheath 1012, as shown in FIGS. 49A, 49B. As shown in FIG. 49A, the silicone sheath 1003 is formed into a U-shape, with one end anchored to an anchor piece 1019 for connection to an adjustable closure 1018, which can be similar to the closure 706 shown in FIG. 34A. The SMA wire 1001 is anchored to an underlying fabric layer, such as fabric 1006 in FIG. 47, by anchors 1002. The silicone sheath 1003 is affixed to a less pliant tubing, such as nylon tubing 1012, by fittings 1014. The fittings 1014 may be in the form of shrink tubing that is heat shrunk around both tubings 1003, 1012 to secure fix the tubing together. The fittings 1014 may be fastened to a separate anchor strip 1016 that is fastened to the underlying fabric. The same fitting can be used to affix the nylon tubing to the SMA wire anchors 1002, as shown in FIG. 49B. The nylon tubing has a low friction inner surface but is otherwise generally less pliant than the silicone tubing 1003. This combination of tubing focuses the stroke of the compression device to the silicone tubing 1003, thereby providing a well-controlled region of compression.

The voltage duty cycle can be regulated to regulate not only the energy entering the system but also the energy leaving the system. Reducing the thickness of the sheath 1003 increase the thermal energy expelled from the wire arrangements 1000, which thermal energy manifests in an increase in temperature of the compression device to the user, which may be desirable. Alternatively, increasing the thickness of the sheath 1003 decreases the thermal energy exiting the wire arrangement so that the compression device no longer performs like a heating pad. However, limiting the thermal energy leaving the wire arrangement means that more heat is retained by the SMA wire, which in turn means that less additional energy is required to keep the SMA wire arrangement at its level of contraction.

In some cases it is desirable to cycle the wire arrangement between the contracted condition (i.e., Austenitic phase of the SMA wire) and the relaxed condition (i.e., Martinsitic phase of the SMA wire). In this case a balance must be maintained between the input energy and the thermal energy that is either stored/retained by the sheath and the energy that is dissipated through the sheath. In this case, shorter input pulses of higher voltages may be preferred, with the understanding that the higher input voltages produces faster actuation speeds for the device. Tight control can be achieved by monitoring the current in the SMA wire 1001. For example, a Nitinol wire of 20 cm (50 inch) length and 0,006 cm (0.015 inch) diameter in a silicone sheath pressed against the body can require 21 volts to heat the wire to full phase change in about 2.5 seconds. The initial current pulse is about 1.7 amps and then peaks to about 2 amps after the phase change. If there is a positive heat transfer from the wire arrangement to the body a steady state condition can be achieved in which the energy applied to the wire arrangement equals the energy exiting. When the current can be increased no further, the wire temperature can be maintained at just above the Austenitic state nearly indefinitely. On the other hand, if the heat transfer is negative, meaning that heat is transferred from the person's body to the wire arrangement, the wire temperature can rise quickly once the SMA wire reaches its Austenitic phase. It is at this point that the controller can turn off the voltage applied to the wire arrangement to regulate both the contraction of the wire arrangement and the heat transfer condition between the user and the wire arrangement. In addition, at this point the voltage may continue to be applied to the wire arrangement in order to achieve a particular heating effect during a compression cycle, being careful to avoid overheating the SMA wire. In a typical case the SMA wire can be damaged if its temperature exceeds 130°C.

In accordance with one aspect of the present device, the device controller monitors the current flow through the SMA wires, and in particular monitors the change in slope of the current pulse. If the slope falls below a threshold the electrical current is shut off to the SMA wire. In one example, that threshold is when the slope drops below 0.1 amp per second increase. The current can be measured by Hall-effect sensors or similar means, and the slope can be calculated in real-time by the controller and stored throughout each actuation cycle for continuous evaluation. As a fail-safe, a maximum voltage pulse on-time can be established, such as 2.5 seconds for a given actuation cycle. It is contemplated that the actuation time for a compression device according to the present disclosure may require a longer cycle time when the device is first turned on because the SMA wires are at ambient temperature well below the phase transition temperature. It is further contemplated that with each successive actuation cycle the actuation time will decrease because the SMA wire has already reached the transition temperature and the ability of the sheath to shed thermal energy gradually decreases as the user's body temperature inside the compression device increases. Under these conditions it is then necessary to shorten the voltage pulses to prevent the SMA wire from over-heating. Under certain conditions, extended "rest" for the compression device and wire arrangements maybe necessary to allow the SMA to cool below the transition temperature and below the Martinsitic finish temperature. Thus, in some cases this rest period can be 60-90 seconds.

In one method for controlling the current flow through the SMA wires, the length of the wires can be continuously sampled and then the current shut off to the wires when they reach their fully transformed length. Thus, in one aspect, a time domain reflectometer (TDR) or a frequency domain reflectometer (FDR) can be used to measure the initial length of the SMA wire and then to continuously sample the wire length. The TDR or FDR can be configured to signal the device controller to turn the power off when the wire shrinks to its full transformation length. In another approach, a mechanical switch may be provided in lieu of the TDR or FDR. Thus, as shown in FIG. 50, the SMA wire 1020 may be anchored to an end connector 1024 and slidably disposed within a sheath 1022. Electrical contacts 1025 are mounted to the end connector 1024, and contacts 1026 are mounted to the end of the sheath 1022, with wires 1027 communicating with the device controller. When the SMA wire 1020 shrinks to its desired length, the contracting wire draws the end connector 1024 toward the sheath 1022 until the electrical contacts 1025, 1026 meet. When the contacts meet, the wire circuit 1027 is closed to send a signal to the device controller which responds accordingly to stop current flow to the SMA wire 1020.

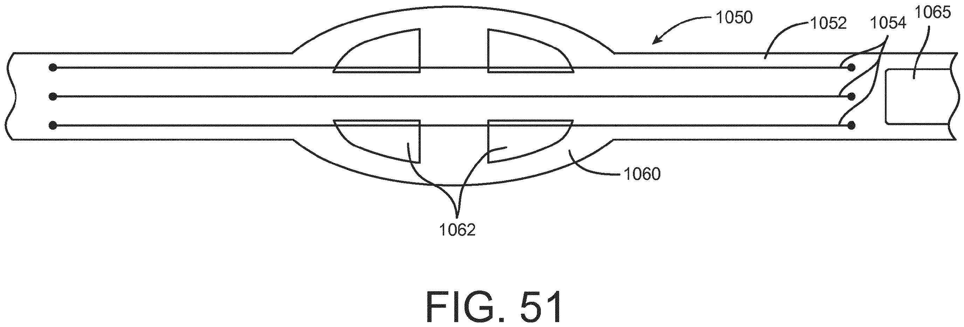

In a further embodiment, a compression device 1050 combines the active compression features of the SMA wire devices described above with an electrical stimulation device. Thus, as shown in FIG. 51, a compression device 1050 can include a base 1052 configured to be worn on the user, such as in the manner of any of the compression devices described herein. The device is provided with SMA wires 1054 that are powered and controlled by a device controller 1065, again in any of the manners described herein. The base 1052 includes a stimulation region 1060 on which is mounted a plurality of electrical stimulation devices 1062. In one embodiment, the devices are TENS- transcutaneous electrical nerve stimulation - units. The TENS units 1062 are connected to the device controller 1065 which is configured to activate and control the TENS units according to a desired protocol, such as by controlling pulse width, frequency and intensity of the electrical signal emanating from the devices. The compression device 1050 can position at least one SMA wire 1054 over each TENS unit 1062 to help press and maintain the TENS unit in direct contact with the user's skin, which is necessary for the TENS units to operate. Since the SMA wire electrical requirements and duty cycle may differ from the TENS unit duty cycle and electrical requirements, the TENS units 1062 are separately wired to the device controller 1065.

The device controller 1065 can be configured to modulate the SMA wires and TENS units according to a desired protocol. For instance, in one protocol the SMA wires 1054 are activated to provide compression for a fixed period, say four seconds, and then while the SMA wires are relaxed the TENS units 1062 are activated for another four seconds, followed by four seconds of rest. This cycle can be repeated as long as desired, and may be accompanied by the addition of heat to the compression and/or TENS activation parts of the cycle.

## Claims

1. A compression device (10, 600, 700, 740, 760, 780, 790, 795, 800, 810, 920, 935, 937, 1010, 1050) for applying controllable compression to a portion of the anatomy of a user, comprising:
a panel formed of a wearable material, the panel (12, 602, 610, 704, 811-816) sized and configured to be applied to a portion of the anatomy of the user, the panel having opposite ends configured to oppose each other when the panel is applied to the portion of the anatomy;
a plurality of shape memory wires ( 14, 705, 910, 915, 936, 938, 1001, 1020, 1054) supported by the panel between said opposite ends and configured to apply a compressive force to the portion of the anatomy of the user;
a pre-tensioning apparatus (62, 620, 706, 711, 820) connectable between said opposite ends when the panel is applied to the portion of the anatomy, the pre-tensioning apparatus operable to draw the opposite ends toward each other to thereby apply an initial tension to the panel and/or the plurality of shape-memory wires; and
a controller (24, 66, 1065) configured to selectively actuate one or more of the plurality of shape-memory wires to reduce the effective length of the wires and thus the panel about the portion of the anatomy, to thereby apply pressure to the portion of the anatomy of the user, **characterized in that**
each of said plurality of shape-memory wires is disposed within an elastomeric sheath (714, 1003, 1022),
wherein the sheath (714, 1003, 1022) is formed of silicone or a polyurethane material, wherein the sheath material is doped with a thermally conductive or thermally insulative material to adjust the overall conductivity of the sheath(714, 1003, 1022), to have a thermal conductivity sufficient to help the shape memory wire (14, 705, 910, 915, 936, 938, 1001, 1020, 1054) maintain its temperature upon contraction with only limited additional energy input required to maintain the same level of compression.

2. The compression device of claim 1, wherein the sheath material is doped with air bubbles, glass beads or diamond dust.

3. The compression device of claim 1, wherein the shape memory wire is slidably disposed within the sheath.

4. The compression device of claim 1, wherein the elastomeric sheath is coated onto the shape memory wire.

5. The compression device of claim 1, wherein the elastomeric sheath is anchored to the panel.

6. The compression device of claim 1, wherein:
the device is provided with an electrical power supply (30, 70),; and
the controller is configured to selectively apply a current from the power supply to one or more of the plurality of shape-memory wires.

7. The compression device of claim 6, wherein the power supply and controller are separate from the panel and wherein the plurality of shape-memory wires are connected to one or more cables (23, 622, 703, 706, 713, 716) and the controller includes one or more mating cables for electrical connection to the one or more cables.

8. The compression device of claim 1, wherein the pre-tensioning device includes:
at least one connector (708, 709) affixed to one end of the panel and a corresponding mating connector adapted for releasable engagement to a corresponding one of said at least one connector;
at least one rotary dial (706c, 711, 820) fastened to said opposite end of the panel; and
a cable(703, 706, 713, 716) passing through said at least one rotary dial and one or more of said corresponding connector, said rotary dial configured to reduce the length of the cable between said dial and said corresponding connector, whereby when the corresponding connector is engaged to a corresponding one of said at least one connector at least a portion of the panel adjacent said rotary dial is pre-tensioned,
wherein said cable passing through said at least one rotary dial is preferably one of the plurality of shape-memory wires.

9. The compression device of claim 1, wherein the device (780, 784) is configured to be worn on the user's shoulder, and includes:
a first portion (781a, 786a) configured to wrap around the upper arm;
a second portion (781b, 786b) that extends across the chest and upper back of the user; and
a number of straps (782, 785) that extend from the second portion and wrap around the chest and back of the user.

10. The compression device of claim 1, wherein the device is configured as a vest (790) adapted to be worn about the torso or lower back of the user, or, wherein the device is configured as athletic shorts (795), belt or wrap apparatus to apply compression to the hip and/or IT band and/or gluteus muscles of the user.

11. The compression device of claim 1, wherein the controller includes a microcontroller (24, 66) configured to apply current simultaneously to selected ones of said plurality of shape-memory wires sufficient to achieve a 2-15% reduction in length or shrinkage of the wire over a predetermined time period.

12. The compression device (935, 937) of claim 1, wherein the plurality of shape memory wires (936, 938) are arranged in a sinusoidal pattern on the panel in a prestressed condition and are configured to be substantially linear in a stressed condition prior to actuation by the controller.

13. The compression device of claim 1,
wherein the controller is operable to measure the current flow through each shape memory wire during actuation, the change in current flow through the wire and the slope of the change in current flow, and is further operable to shut off the current to a corresponding wire if the change in slope of the current falls below a threshold, preferably that the slope is less than 0.1 amps per second increase.

14. The compression device of claim 1,
wherein the controller configured to measure the length of a wire during actuation and is operable to shut off the current to a corresponding wire when the measured length reaches a threshold,
wherein the controller preferably includes a time domain reflectometer or a frequency domain reflectometer operable to measure the length of the corresponding wire.

15. The compression device of claim 1, comprising:
a transcutaneous electrical nerve stimulation (TENS) unit supported by the panel for contact with the user's skin, wherein the controller is further configured to selectively actuate the TENS unit, preferably to alternate actuation of the wires and the TENS unit relative to each other.

## Patentansprüche

1. Kompressionsvorrichtung (10, 600, 700, 740, 760, 780, 790, 795, 800, 810, 920, 935, 937, 1010, 1050) zum Ausüben von steuerbarer Kompression auf einen Anteil der Anatomie eines Anwenders, umfassend:
eine Stoffbahn, die aus einem tragbaren Material gebildet ist, wobei die Stoffbahn (12, 602, 610, 704, 811-816) bemessen und ausgestaltet ist, um auf einen Anteil der Anatomie des Anwenders aufgebracht zu werden, wobei die Stoffbahn gegenüber liegende Enden aufweist, die ausgestaltet sind, um einander gegenüber zu liegen, wenn die Stoffbahn auf den Anteil der Anatomie aufgebracht ist;
eine Vielzahl von Formgedächtnisdrähten (14, 705, 910, 915, 936, 938, 1001, 1020, 1054), die durch die Stoffbahn zwischen den gegenüber liegenden Enden getragen und ausgestaltet sind, um eine Kompressionskraft auf den Anteil der Anatomie des Anwenders auszuüben;
einen Vorspannapparat (62, 620, 706, 711, 820), der zwischen den gegenüber liegenden Enden verbindbar ist, wenn die Stoffbahn auf den Anteil der Anatomie aufgebracht ist, wobei der Vorspannapparat funktional ist, um die gegenüber liegenden Enden aufeinander zu zu ziehen, um dadurch eine Anfangsspannung auf die Stoffbahn und/oder die Vielzahl von Formgedächtnisdrähten auszuüben; und
eine Steuerung (24, 66, 1065), die ausgestaltet ist, um selektiv einen oder mehrere von der Vielzahl der Formgedächtnisdrähte zu betätigen, um die effektive Länge der Drähte und somit der Stoffbahn um den Anteil der Anatomie zu reduzieren, um dadurch Druck auf den Anteil der Anatomie des Anwenders auszuüben,
**dadurch gekennzeichnet, dass**
jeder von der Vielzahl der Formgedächtnisdrähte innerhalb einer elastomeren Hülle (714, 1003, 1022) angeordnet ist,
wobei die Hülle (714, 1003, 1022) aus Silikon oder einem Polyurethanmaterial gebildet ist, wobei das Hüllenmaterial mit einem wärmeleitfähigen oder wärmeisolierenden Material dotiert ist, um die Gesamtleitfähigkeit der Hülle (714, 1003, 1022) anzupassen, um über eine ausreichende Wärmeleitfähigkeit zu verfügen, um den Formgedächtnisdraht (14, 705, 910, 915, 936, 938, 1001, 1020, 1054) darin zu unterstützen, seine Temperatur bei Kontraktion aufrecht zu erhalten, wobei nur begrenzte zusätzliche Energiezufuhr erforderlich ist, um das gleiche Kompressionsniveau aufrecht zu erhalten.

2. Kompressionsvorrichtung nach Anspruch 1, wobei das Hüllenmaterial mit Luftbläschen, Glasperlen oder Diamantstaub dotiert ist.

3. Kompressionsvorrichtung nach Anspruch 1, wobei der Formgedächtnisdraht gleitfähig innerhalb der Hülle angeordnet ist.

4. Kompressionsvorrichtung nach Anspruch 1, wobei die elastomere Hülle als Beschichtung auf den Formgedächtnisdraht aufgebracht ist.

5. Kompressionsvorrichtung nach Anspruch 1, wobei die elastomere Hülle an der Stoffbahn verankert ist.

6. Kompressionsvorrichtung nach Anspruch 1, wobei:
die Vorrichtung mit einer elektrischen Leistungsversorgung (30, 70) ausgestattet ist; und
die Steuerung ausgestaltet ist, um selektiv einen Strom von der Leistungsversorgung an einen oder mehrere von der Vielzahl der Formgedächtnisdrähte anzulegen.

7. Kompressionsvorrichtung nach Anspruch 6, wobei die Leistungsversorgung und die Steuerung von der Stoffbahn getrennt vorliegen, und wobei die Vielzahl der Formgedächtnisdrähte mit einem oder mehreren Kabeln (23, 622, 703, 706, 713, 716) verbunden ist und die Steuerung ein oder mehrere Paarungskabel zur elektrischen Verbindung mit dem einen oder den mehreren Kabeln einschließt.

8. Kompressionsvorrichtung nach Anspruch 1, wobei die Vorspannvorrichtung einschließt:
mindestens einen Verbinder (708, 709), der an einem Ende der Stoffbahn fixiert ist, und einen korrespondierenden Paarungsverbinder, der zum lösbaren Eingriff mit einem korrespondierenden des mindestens einen Verbinders eingerichtet ist;
mindestens einen Drehregler (706c, 711, 820), der an dem gegenüber liegenden Ende der Stoffbahn befestigt ist; und
ein Kabel (703, 706, 713, 716), das durch den mindestens einen Drehregler und einen oder mehrere der korrespondierenden Verbinder geführt ist, wobei der Drehregler ausgestaltet ist, um die Länge des Kabels zwischen dem Regler und dem korrespondierenden Verbinder zu reduzieren, wobei, wenn der korrespondierende Verbinder in Eingriff mit einem korrespondierenden des mindestens einen Verbinders kommt, mindestens ein Anteil der Stoffbahn benachbart zu dem Drehregler vorgespannt wird,
wobei das durch den mindestens einen Drehregler geführte Kabel vorzugsweise einer von der Vielzahl der Formgedächtnisdrähte ist.

9. Kompressionsvorrichtung nach Anspruch 1, wobei die Vorrichtung (780, 784) ausgestaltet ist, um auf der Schulter des Anwenders getragen zu werden, und einschließt:
einen ersten Anteil (781a, 786a), der ausgestaltet ist, um um den Oberarm gewickelt zu werden;
einen zweiten Anteil (781b, 786b), der sich über die Brust und den oberen Rücken des Anwenders erstreckt; und
eine Anzahl von Riemen (782, 785), die sich von dem zweiten Anteil erstrecken und um die Brust und den Rücken des Anwenders gewickelt sind.

10. Kompressionsvorrichtung nach Anspruch 1, wobei die Vorrichtung als Weste (790) ausgestaltet ist, die eingerichtet ist, um um den Torso oder unteren Rücken des Anwenders getragen zu werden, oder wobei die Vorrichtung als sportive Shorts (795), Gürtel oder Wickelapparat ausgestaltet ist, um Kompression auf die Hüfte und/oder das IT-Band und/oder die Gluteus-Muskeln des Anwenders auszuüben.

11. Kompressionsvorrichtung nach Anspruch 1, wobei die Steuerung einen Mikrocontroller (24, 66) einschließt, der ausgestaltet ist, um simultan Strom an ausgewählte aus der Vielzahl der Formgedächtnisdrähte anzulegen, der ausreicht, um eine Längenreduktion oder Schrumpfung des Drahts von 2 bis 15 % über einen vorbestimmten Zeitraum zu erreichen.

12. Kompressionsvorrichtung (935, 937) nach Anspruch 1, wobei die Vielzahl der Formgedächtnisdrähte (936, 938) in einem Sinusmuster auf der Stoffbahn in einem vorgespannten Zustand angeordnet und ausgestaltet ist, um in dem gespannten Zustand, bevor die Steuerung betätigt wird, im Wesentlichen linear zu sein.

13. Kompressionsvorrichtung nach Anspruch 1,
wobei die Steuerung funktional ist, um den Stromfluss durch jeden Formgedächtnisdraht während der Betätigung zu messen, die Änderung des Stromflusses durch den Draht hindurch und die Neigung der Änderung des Stromflusses zu messen, und des Weiteren funktional ist, um den Strom zu einem entsprechenden Draht abzuschalten, falls die Änderung der Neigung des Stroms unter einen Schwellenwert sinkt, wobei die Neigung vorzugsweise weniger als 0,1 Ampere pro Sekunde Anstieg beträgt.

14. Kompressionsvorrichtung nach Anspruch 1,
wobei die Steuerung ausgestaltet ist, um die Länge eines Drahts während der Betätigung zu messen, und funktional ist, um den Strom zu einem korrespondierenden Draht abzuschalten, wenn die gemessene Länge einen Schwellenwert erreicht,
wobei die Steuerung vorzugsweise ein Zeitdomänenreflektometer oder ein Frequenzdomänenreflektometer einschließt, der funktional wist, um die Länge des korrespondierenden Drahts zu messen.

15. Kompressionsvorrichtung nach Anspruch 1, umfassend:
eine transkutane elektrische Nervenstimulations- (TENS)-Einheit, die durch die Stoffbahn getragen wird, um in Kontakt mit der Haut des Anwenders zu sein, wobei die Steuerung des Weiteren ausgestaltet ist, um die TENS-Einheit selektiv zu betätigen, und um vorzugsweise die Betätigung der Drähte und der TENS-Einheit relativ zueinander zu alternieren.

## Revendications

1. Dispositif de compression (10, 600, 700, 740, 760, 780, 790, 795, 800, 810, 920, 935, 937, 1010, 1050) pour appliquer une compression contrôlable à une partie de l'anatomie d'un utilisateur, comprenant :
un panneau formé d'un matériau pouvant être porté, le panneau (12, 602, 610, 704, 811-816) étant dimensionné et configuré pour être appliqué à une partie de l'anatomie de l'utilisateur, le panneau ayant des extrémités opposées configurées pour être opposées l'une à l'autre lorsque le panneau est appliqué à la partie de l'anatomie ;
une pluralité de fils à mémoire de forme (14, 705, 910, 915, 936, 938, 1001, 1020, 1054) supportés par le panneau entre lesdites extrémités opposées et configurés pour appliquer une force de compression à la partie de l'anatomie de l'utilisateur ;
un appareil de pré-tension (62, 620, 706, 711, 820) pouvant être connecté entre lesdites extrémités opposées lorsque le panneau est appliqué à la partie de l'anatomie, l'appareil de pré-tension pouvant fonctionner pour tirer les extrémités opposées l'une vers l'autre pour ainsi appliquer une tension initiale au panneau et/ou à la pluralité de fils à mémoire de forme ; et
un contrôleur (24, 66, 1065) configuré pour actionner sélectivement un ou plusieurs fils de la pluralité de fils à mémoire de forme afin de réduire la longueur effective des fils et donc du panneau autour de la partie de l'anatomie, pour ainsi appliquer une pression à la partie de l'anatomie de l'utilisateur, **caractérisé en ce que**
chacun de ladite pluralité de fils à mémoire de forme est disposé à l'intérieur d'une gaine élastomère (714, 1003, 1022),
dans lequel la gaine (714, 1003, 1022) est formée de silicone ou d'un matériau de polyuréthane, où le matériau de gaine est dopé avec un matériau thermiquement conducteur ou thermiquement isolant afin d'ajuster la conductivité globale de la gaine (714, 1003, 1022), pour avoir une conductivité thermique suffisante pour aider le fil à mémoire de forme (14, 705, 910, 915, 936, 938, 1001, 1020, 1054) à maintenir sa température lors de la contraction avec seulement un apport d'énergie supplémentaire limité requis pour maintenir le même niveau de compression.

2. Dispositif de compression de la revendication 1, dans lequel le matériau de gaine est dopé avec des bulles d'air, des billes de verre ou de la poussière de diamant.

3. Dispositif de compression de la revendication 1, dans lequel le fil à mémoire de forme est disposé de manière coulissante à l'intérieur de la gaine.

4. Dispositif de compression de la revendication 1, dans lequel la gaine élastomère est appliquée en revêtement sur le fil à mémoire de forme.

5. Dispositif de compression de la revendication 1, dans lequel la gaine élastomère est ancrée au panneau.

6. Dispositif de compression de la revendication 1, dans lequel :
le dispositif est pourvu d'une alimentation électrique (30, 70) ; et
le contrôleur est configuré pour appliquer sélectivement un courant provenant de l'alimentation électrique à un ou plusieurs fils de la pluralité de fils à mémoire de forme.

7. Dispositif de compression de la revendication 6, dans lequel l'alimentation électrique et le contrôleur sont séparés du panneau et dans lequel la pluralité de fils à mémoire de forme sont connectés à un ou plusieurs câbles (23, 622, 703, 706, 713, 716) et le contrôleur comprend un ou plusieurs câbles d'accouplement pour une connexion électrique au ou aux plusieurs câbles.

8. Dispositif de compression de la revendication 1, dans lequel le dispositif de pré-tension comprend :
au moins un connecteur (708, 709) apposé sur une extrémité du panneau et un connecteur d'accouplement correspondant adapté pour un engagement libérable avec un connecteur correspondant dudit au moins un connecteur ;
au moins un cadran rotatif (706c, 711, 820) fixé à ladite extrémité opposée du panneau ; et
un câble (703, 706, 713, 716) passant à travers ledit au moins un cadran rotatif et un ou plusieurs dudit connecteur correspondant, ledit cadran rotatif étant configuré pour réduire la longueur du câble entre ledit cadran et ledit connecteur correspondant, moyennant quoi, lorsque le connecteur correspondant est engagé avec un connecteur correspondant dudit au moins un connecteur, au moins une partie du panneau adjacente audit cadran rotatif est pré-tendue,
dans lequel ledit câble traversant ledit au moins un cadran rotatif est de préférence l'un de la pluralité de fils à mémoire de forme.

9. Dispositif de compression de la revendication 1, dans lequel le dispositif (780, 784) est configuré pour être porté sur l'épaule de l'utilisateur, et comprend :
une première partie (781a, 786a) configurée pour s'enrouler autour du bras supérieur ;
une deuxième partie (781b, 786b) qui s'étend sur la poitrine et le haut du dos de l'utilisateur ; et
un certain nombre de sangles (782, 785) qui s'étendent depuis la deuxième partie et s'enroulent autour de la poitrine et du dos de l'utilisateur.

10. Dispositif de compression de la revendication 1, dans lequel le dispositif est configuré comme un gilet (790) adapté pour être porté autour du torse ou du bas du dos de l'utilisateur, ou, dans lequel le dispositif est configuré comme un short de sport (795), une ceinture ou appareil d'enveloppement pour appliquer une compression sur la hanche et/ou la bande ilio-tibiale et/ou les muscles fessiers de l'utilisateur.

11. Dispositif de compression de la revendication 1, dans lequel le contrôleur comprend un microcontrôleur (24, 66) configuré pour appliquer un courant simultanément à des fils sélectionnés de ladite pluralité de fils à mémoire de forme suffisant pour obtenir une réduction de 2 à 15% de la longueur ou un rétrécissement du fil pendant une période de temps prédéterminée.

12. Dispositif de compression (935, 937) de la revendication 1, dans lequel la pluralité de fils à mémoire de forme (936, 938) sont agencés selon un motif sinusoïdal sur le panneau dans une condition de précontrainte et sont configurés pour être sensiblement linéaires dans une condition de contrainte avant l'actionnement par le contrôleur.

13. Dispositif de compression de la revendication 1,
dans lequel le contrôleur peut fonctionner pour mesurer le flux de courant à travers chaque fil à mémoire de forme pendant l'actionnement, le changement de flux de courant à travers le fil et la pente du changement de flux de courant, et peut en outre fonctionner pour couper le courant vers un fil correspondant si le changement de pente du courant tombe en dessous d'un seuil, de préférence lorsque la pente représente une augmentation de moins de 0,1 ampère par seconde.

14. Dispositif de compression de la revendication 1,
dans lequel le contrôleur est configuré pour mesurer la longueur d'un fil pendant l'actionnement et peut fonctionner pour couper le courant vers un fil correspondant lorsque la longueur mesurée atteint un seuil,
dans lequel le contrôleur comprend de préférence un réflectomètre dans le domaine temporel ou un réflectomètre dans le domaine fréquentiel pouvant fonctionner pour mesurer la longueur du fil correspondant.

15. Dispositif de compression de la revendication 1, comprenant :
une unité de stimulation nerveuse électrique transcutanée (TENS) supportée par le panneau pour un contact avec la peau de l'utilisateur, où le contrôleur est en outre configuré pour actionner sélectivement l'unité TENS, de préférence pour alterner l'actionnement des fils et de l'unité TENS l'un par rapport à l'autre.
